# EUROPÄISCHE PATENTSCHRIFT

(11) **EP 1 228 235 B1**
(45) Veröffentlichungstag und Bekanntmachung des Hinweises auf die Patenterteilung: **25.08.2004**
(21) Anmeldenummer: 00979529.5
(22) Anmeldetag: 03.11.2000
(51) Int. Cl.: C12N 15/87, C07K 19/00

(54) **VERFAHREN ZUM TRANSFER VON MOLEKULAREN SUBSTANZEN MIT PROKARYONTISCHEN NUKLEINSAURE-BINDENDEN PROTEINEN**
METHOD FOR TRANSFER OF MOLECULAR SUBSTANCES WITH PROKARYONTIC NUCLEIC ACID-BINDING PROTEINS
PROCEDE DE TRANSFERT DE SUBSTANCES MOLECULAIRES AU MOYEN DE PROTEINES PROCARYOTIQUES LIANT DES ACIDES NUCLEIQUES

(30) Priorität: 03.11.1999 DE 19952983
(43) Veröffentlichungstag der Anmeldung: 07.08.2002
(73) Patentinhaber: ACGT Progenomics AG, 06120 Halle (Saale) (DE)
(72) Erfinder: BÖHM, Gerald, 06114 Halle (Saale) (DE); ESSER, Dirk, Cherry Hinton, Cambridge CB1 9EW (GB)
(74) Vertreter: Reinhard - Skuhra - Weise & Partner
(86) Internationale Anmeldenummer: PCT/EP2000/010875
(87) Internationale Veröffentlichungsnummer: WO 2001/032900

(56) Entgegenhaltungen:
- US-A- 4 179 337
- US-A- 5 965 404
- ZIEMIENOWICZ A. ET AL.: "Import of DNA into mammalian nuclei by proteins originating from a plant pathogenic bacterium." PROCEEDINGS OF THE NATIONAL ACADEMY OF SCIENCES OF THE UNITED STATES, Bd. 96, Nr. 7, 30. März 1999 (1999-03-30), Seiten 3729-3733, XP002162393 ISSN: 0027-8424
- ESSER D. ET AL.: "The HU protein from Thermotoga maritima: Recombinant expression, purification and physicochemical characterization of an extremely hyperthermophilic DNA-binding protein." JOURNAL OF MOLECULAR BIOLOGY, Bd. 291, Nr. 5, Seiten 1135-1146, XP002162394 ISSN: 0022-2836
- FRITZ J. D. ET AL.: "GENE TRANSFER INTO MAMMALIAN CELLS USING HISTONE-CONDENSED PLASMID DNA" HUMAN GENE THERAPY, Bd. 7, 1. August 1996 (1996-08-01), Seiten 1395-1404, XP002058321 ISSN: 1043-0342
- BOULIKAS T. ET AL.: "HISTONES, PROTAMINE, AND POLYLYSINE BUT NOT POLY(E:K) ENHANCE TRANSFECTION EFFICIENCY" INTERNATIONAL JOURNAL OF ONCOLOGY, Bd. 10, Nr. 2, 1. Februar 1997 (1997-02-01), Seiten 317-322, XP002058322 ISSN: 1019-6439
- BRANDEN L. J. ET AL.: "PEPTIDE NUCLEIC ACID-NUCLEAR LOCALIZATION SIGNAL FUSION THAT MEDIATES NUCLEAR TRANSPORT OF DNA" NATURE BIOTECHNOLOGY, Bd. 17, Nr. 8, August 1999 (1999-08), Seiten 784-787, XP000940836 ISSN: 1087-0156
- ELLIOTT G. ET AL.: "INTERCELLULAR TRAFFICKING AND PROTEIN DELIVERY BY A HERPESVIRUS STRUCTURAL PROTEIN" CELL, Bd. 88, 24. Januar 1997 (1997-01-24), Seiten 223-233, XP000961185 ISSN: 0092-8674
- BIAN J. ET AL.: "Nuclear translocation of HIV-1 matrix protein P17: The use of Aequorea victoria green fluorescence protein in protein tagging and tracing." FASEB JOURNAL, Bd. 9, Nr. 6, 1995, Seite A1279 XP002162395 Annual Meeting of the American Society for Biochemistry and Molecular Biology; San Francisco, California, USA; May 21-25, 1995 ISSN: 0892-6638
- ESSER D. ET AL.: "A hyperthermostable bacterial histone-like protein as an efficient mediator for transfection of eukaryotic cells." NATURE BIOTECHNOLOGY, Bd. 18, Nr. 11, November 2000 (2000-11), Seiten 1211-1213, XP002162396 ISSN: 1087-0156

### SEQUENZPROTOKOLL

<110> ACGT ProGenomics AG
<120> Verfahren zum Transfer von molekularen Substanzen mit prokaryontischen Nukleinsäure-bindenden Proteinen
<130> P12603
<140>
   <141>
<150> DE - 199 52 983.3
   <151> 1999-11-03
<160> 7
<170> PatentIn Ver. 2.1
<210> 1
   <211> 270
   <212> DNA
   <213> Thermotoga maritima
<220>
   <221> CDS
   <222> (1) .. (270)
   <223> Kodierende Gensequenz von TmHU
<400> 1
   atg aac aaa aaa gaa ctg atc gac agg gtg gcg aag aaa gca ggt gcg    48
   Met Asn Lys Lys Glu Leu Ile Asp Arg Val Ala Lys Lys Ala Gly Ala
    1               5                  10                  15
   aag aaa aag gat gta aaa ttg att ctc gac acc atc ctt gaa acg atc    96
   Lys Lys Lys Asp Val Lys Leu Ile Leu Asp Thr Ile Leu Glu Thr Ile
               20                  25                  30
   aca gaa gct ctc gca aag ggt gaa aag gtt cag atc gtt gga ttc gga   144
   Thr Glu Ala Leu Ala Lys Gly Glu Lys Val Gln Ile Val Gly Phe Gly
           35                  40                  45
   agc ttc gaa gtg agg aag gcc gct gca aga aaa ggc gtg aat cct cag   192
   Ser Phe Glu Val Arg Lys Ala Ala Ala Arg Lys Gly Val Asn Pro Gln
       50                  55                  60
   aca aga aaa ccc atc acc att ccc gaa aga aag gtc ccg aag ttc aaa   240
   Thr Arg Lys Pro Ile Thr Ile Pro Glu Arg Lys Val Pro Lys Phe Lys
   65                  70                  75                  80
   ccc gga aaa gcc ctc aaa gag aag gtc aag                           270
   Pro Gly Lys Ala Leu Lys Glu Lys Val Lys
                   85                  90
<210> 2
   <211> 90
   <212> PRT
   <213> Thermotoga maritima
<400> 2
   Met Asn Lys Lys Glu Leu Ile Asp Arg Val Ala Lys Lys Ala Gly Ala
   1               5                   10                  15
   Lys Lys Lys Asp Val Lys Leu Ile Leu Asp Thr Ile Leu Glu Thr Ile
               20                  25                  30
   Thr Glu Ala Leu Ala Lys Gly Glu Lys Val Gln Ile Val Gly Phe Gly
           35                  40                  45
   Ser Phe Glu Val Arg Lys Ala Ala Ala Arg Lys Gly Val Asn Pro Gln
       50                  55                  60
   Thr Arg Lys Pro Ile Thr Ile Pro Glu Arg Lys Val Pro Lys Phe Lys
   65                  70                  75                  80
   Pro Gly Lys Ala Leu Lys Glu Lys Val Lys
                   85                  90
<210> 3
   <211> 468
   <212> DNA
   <213> Künstliche Sequenz
<220>
   <223> Beschreibung der künstlichen Sequenz: Fusion von EGF mit TmHU
<220>
   <221> CDS
   <222> (1)..(468)
   <223> Kodierende Sequenz von TmHU-EGF
<220>
<221> misc_feacure
   <222> (1)..(267)
   <223> TmHU-Teil des Fusionsproteins
<220>
   <221> misc_feature
   <222> (289)..(468)
   <223> EGF-Teil des Fusionsproteins
<400> 3
   atg aac aaa aaa gaa ctg atc gac agg gtg gcg aag aaa gca ggt gcg    48
   aag aaa aag gat gta aaa ttg att ctc gac acc atc ctt gaa acg atc    96
   aca gaa gct ctc gca aag ggt gaa aag gtt cag atc gtt gga ttc gga    144
   agc ttc gaa gtg agg aag gcc gct gca aga aaa ggc gtg aat cct cag    192
   aca aga aaa ccc atc acc att ccc gaa aga aag gtc ccg aag ttc aaa    240
   ccc gga aaa gcc ctc aaa gag aag gtc ccg cgg cac tat tcc gta gga    288
   aat agt gac tct gaa tgt ccc ctg tcc cac gat ggg tac tgc ctc cat    336
   gat ggt gtg tgc atg tat att gaa gca ttg gac aag tat gca tgc aac    384
   tgt gtt gtt ggc tac atc ggg gag cga tgt cag tac cga gac ctg aag    432
   tgg tgg gaa ctg ggc cac gct ggc cac ggg ccg cgg                    468
<210> 4
   <211> 156
   <212> PRT
   <213> Künstliche Sequenz
   <223> Beschreibung der künstlichen Sequenz: Fusion von EGF mit TmHU
<400> 4
<210> 5
   <211> 1014
   <212> DNA
   <213> Künstliche Sequenz
<220>
   <223> Beschreibung der künstlichen Sequenz: Fusion von TmHU mit eGFP
<220>
   <221> CDS
   <222> (1)..(1020)
   <223> Kodierende Sequenz von TmHU-GFP
<220>
   <221> misc_feature
   <222> (1)..(267)
   <223> TmHU-Teil des Fusionsproteins
<220>
   <221> misc_feature
   <222> (297)..(1014)
   <223> eGFP-Teil des Fusionsproteins
<400> 5
   atg aac aaa aaa gaa ctg atc gac agg gtg gcg aag aaa gca ggt gcg      48
   Met Asn Lys Lys Glu Leu Ile Asp Arg Val Ala Lys Lys Ala Gly Ala
   1               5                   10                  15
   aag aaa aag gat gta aaa ttg att ctc gac acc atc ctt gaa acg atc      96
   Lys Lys Lys Asp Val Lys Leu Ile Leu Asp Thr Ile Leu Glu Thr Ile
               20                  25                  30
   aca gaa gct ctc gca aag ggt gaa aag gtt cag atc gtt gga ttc gga     144
   Thr Glu Ala Leu Ala Lys Gly Glu Lys Val Gln Ile Val Gly Phe Gly
           35                  40                  45
   agc ttc gaa gtg agg aag gcc gct gca aga aaa ggc gtg aat cct cag     192
   Ser Phe Glu Val Arg Lys Ala Ala Ala Arg Lys Gly Val Asn Pro Gln
       50                  55                  60
   aca aga aaa ccc atc acc att ccc gaa aga aag gtc ccg aag ttc aaa     240
   Thr Arg Lys Pro Ile Thr Ile Pro Glu Arg Lys Val Pro Lys Phe Lys
   65                  70                  75                  80
   ccc gga aaa gcc ctc aaa gag aag gtc ccg cgg gcc cgg gat cca ccg     288
   Pro Gly Lys Ala Leu Lys Glu Lys Val Pro Arg Ala Arg Asp Pro Pro
                   85                  90                  95
   gtc gcc acc atg gtg agc aag ggc gag gag ctg ttc acc ggg gtg gtg     336
   Val Ala Thr Met Val Ser Lys Gly Glu Glu Leu Phe Thr Gly Val Val
               100                 105                 110
   ccc atc ctg gtc gag ctg gac ggc gac gta aac ggc cac aag ttc agc     384
   Pro Ile Leu Val Glu Leu Asp Gly Asp Val Asn Gly His Lys Phe Ser
           115                 120                 125
   gtg tcc ggc gag ggc gag ggc gat gcc acc tac ggc aag ctg acc ctg     432
   Val Ser Gly Glu Gly Glu Gly Asp Ala Thr Tyr Gly Lys Leu Thr Leu
       130                 135                 140
   aag ttc atc tgc acc acc ggc aag ctg ccc gtg ccc tgg ccc acc ctc     480
   Lys Phe Ile Cys Thr Thr Gly Lys Leu Pro Val Pro Trp Pro Thr Leu
   145                 150                 155                 160
   gtg acc acc ctg acc tac ggc gtg cag tgc ttc agc cgc tac ccc gac     528
   Val Thr Thr Leu Thr Tyr Gly Val Gln Cys Phe Ser Arg Tyr Pro Asp
                   165                 170                 175
   cac atg aag cag cac gac ttc ttc aag tcc gcc atg ccc gaa ggc tac     576
   His Met Lys Gln His Asp Phe Phe Lys Ser Ala Met Pro Glu Gly Tyr
               180                 185                 190
   gtc cag gag cgc acc atc ttc ttc aag gac gac ggc aac tac aag acc     624
   Val Gln Glu Arg Thr Ile Phe Phe Lys Asp Asp Gly Asn Tyr Lys Thr
           195                 200                 205
   cgc gcc gag gtg aag ttc gag ggc gac acc ctg gtg aac cgc atc gag     672
   Arg Ala Glu Val Lys Phe Glu Gly Asp Thr Leu Val Asn Arg Ile Glu
       210                 215                 220
   ctg aag ggc atc gac ttc aag gag gac ggc aac atc ctg ggg cac aag     720
   Leu Lys Gly Ile Asp Phe Lys Glu Asp Gly Asn Ile Leu Gly His Lys
   225                 230                 235                 240
   ctg gag tac aac tac aac agc cac aac gtc tat atc atg gcc gac aag     768
   Leu Glu Tyr Asn Tyr Asn Ser His Asn Val Tyr Ile Met Ala Asp Lys
                   245                 250                 255
   cag aag aac ggc atc aag gtg aac ttc aag atc cgc cac aac atc gag     816
   Gln Lys Asn Gly Ile Lys Val Asn Phe Lys Ile Arg His Asn Ile Glu
               260                 265                 270
   gac ggc agc gtg cag ctc gcc gac cac tac cag cag aac acc ccc atc     864
   Asp Gly Ser Val Gln Leu Ala Asp His Tyr Gln Gln Asn Thr Pro Ile
           275                 280                 285
   ggc gac ggc ccc gtg ctg ctg ccc gac aac cac tac ctg agc acc cag     912
   Gly Asp Gly Pro Val Leu Leu Pro Asp Asn His Tyr Leu Ser Thr Gln
       290                 295                 300
   tcc gcc ctg agc aaa gac ccc aac gag aag cgc gat cac atg gtc ctg    960
   Ser Ala Leu Ser Lys Asp Pro Asn Glu Lys Arg Asp His Met Val Leu
   305                 310                 315                 320
   ctg gag ttc gtg acc gcc gcc ggg atc act ctc ggc atg gac gag ctg   1008
   Leu Glu Phe Val Thr Ala Ala Gly Ile Thr Leu Gly Met Asp Glu Leu
                   325                 330                 335
   tac aag                                                           1014
   Tyr Lys
<210> 6
   <211> 338
   <212> PRT
   <213> Künstliche Sequenz
   <223> Beschreibung der künstlichen Sequenz: Fusion von TmHU mit eGFP
<400> 6
   Met Asn Lys Lys Glu Leu Ile Asp Arg Val Ala Lys Lys Ala Gly Ala
   1               5                   10                  15
   Lys Lys Lys Asp Val Lys Leu Ile Leu Asp Thr Ile Leu Glu Thr Ile
               20                  25                  30
   Thr Glu Ala Leu Ala Lys Gly Glu Lys Val Gln Ile Val Gly Phe Gly
           35                  40                  45
   Ser Phe Glu Val Arg Lys Ala Ala Ala Arg Lys Gly Val Asn Pro Gln
       50                  55                  60
   Thr Arg Lys Pro Ile Thr Ile Pro Glu Arg Lys Val Pro Lys Phe Lys
   65                  70                  75                  80
   Pro Gly Lys Ala Leu Lys Glu Lys Val Pro Arg Ala Arg Asp Pro Pro
                   85                  90                  95
   Val Ala Thr Met Val Ser Lys Gly Glu Glu Leu Phe Thr Gly Val Val
               100                 105                 110
   Pro Ile Leu Val Glu Leu Asp Gly Asp Val Asn Gly His Lys Phe Ser
           115                 120                 125
   Val Ser Gly Glu Gly Glu Gly Asp Ala Thr Tyr Gly Lys Leu Thr Leu
       130                 135                 140
   Lys Phe Ile Cys Thr Thr Gly Lys Leu Pro Val Pro Trp Pro Thr Leu
   145 150 155 160
   Val Thr Thr Leu Thr Tyr Gly Val Gln Cys Phe Ser Arg Tyr Pro Asp
   165 170 175
   His Met Lys Gln His Asp Phe Phe Lys Ser Ala Met Pro Glu Gly Tyr
   180 185 190
   Val Gln Glu Arg Thr Ile Phe Phe Lys Asp Asp Gly Asn Tyr Lys Thr
   195 200 205
   Arg Ala Glu Val Lys Phe Glu Gly Asp Thr Leu Val Asn Arg Ile Glu
   210 215 220
   Leu Lys Gly Ile Asp Phe Lys Glu Asp Gly Asn Ile Leu Gly His Lys
   225 230 235 240
   Leu Glu Tyr Asn Tyr Asn Ser His Asn Val Tyr Ile Met Ala Asp Lys
   245 250 255
   Gln Lys Asn Gly Ile Lys Val Asn Phe Lys Ile Arg His Asn Ile Glu
   260 265 270
   Asp Gly Ser Val Gln Leu Ala Asp His Tyr Gln Gln Asn Thr Pro Ile
   275 280 285
   Gly Asp Gly Pro Val Leu Leu Pro Asp Asn His Tyr Leu Ser Thr Gln
   290 295 300
   Ser Ala Leu Ser Lys Asp Pro Asn Glu Lys Arg Asp His Met Val Leu
   305 310 315 320
   Leu Glu Phe Val Thr Ala Ala Gly Ile Thr Leu Gly Met Asp Glu Leu
   325 330 335
   Tyr Lys
<210> 7
   <211> 90
   <212> PRT
   <213> Escherichia coli
<220>
   <221> PEPTIDE
   <222> (1)..(90)
<223> Escherichia coli HU-Protein, Alpha-Untereinheit, GenBank Code 118256
<400> 7
   Met Asn Lys Thr Gln Leu Ile Asp Val Ile Ala Glu Lys Ala Glu Leu
    1               5                  10                  15
   Ser Lys Thr Gln Ala Lys Ala Ala Leu Glu Ser Thr Leu Ala Ala Ile
               20                  25                  30
   Thr Glu Ser Leu Lys Glu Gly Asp Ala Val Gln Leu Val Gly Phe Gly
           35                  40                  45
   Thr Phe Lys Val Asn His Arg Ala Glu Arg Thr Gly Arg Asn Pro Gln
       50                  55                  60
   Thr Gly Lys Glu Ile Lys Ile Ala Ala Ala Asn Val Pro Ala Phe Val
   65                  70                  75                  80
   Ser Gly Lys Ala Leu Lys Asp Ala Val Lys
                   85                  90

## Beschreibung

Die vorliegende Erfindung betrifft ein Verfahren zum Transfer von Nukleinsäuren und/oder Nukleinsäureanaloga, und/oder Nukleinsäuren und/oder Nukleinsäureanaloga und/oder Aminosäuren enthaltenden Substanzen, insbesondere von Nukleinsäuren wie DNA in Form von Plasmiden, in prokaryontische oder eukaryontische Zellen.

### Gebiet der Erfindung und Stand der Technik

Transfektion ist das Einbringen von Nukleinsäuren wie beispielsweise Nukleotiden, Antisense-RNA, Ribozymen, oder insbesondere DNA in Form von Plasmiden, Chromosomen, oder Chromosomfragmenten zur Expression eines Gens in Zellen. Der Begriff *Transfer* ist hierzu analog, wobei im allgemeinen Sprachgebrauch die Bezeichung *Transfektion* insbesondere für Gentransfer verwendet wird. Neben dem Transfer von Nukleinsäuren ist auch der gerichtete und effiziente Transfer weiterer Wirkstoffe, wie beispielsweise Proteine, Peptide, Arznei-Wirkstoffe und andere molekulare Substanzen von großem Interesse. Der Transferprozeß besitzt wesentliche Bedeutung sowohl für die biomedizinische Grundlagenforschung als auch für die pharmazeutisch-biotechnologische Industrie. Einige Proteine, darunter therapeutisch relevante, werden nur bei einer Herstellung in eukaryontischen Zellen korrekt prozessiert, da die Modifikationsmaschinerie, die ein Protein nach der Translation noch verändert, in prokaryontischen Organismen zum großen Teil nicht vorhanden oder erheblich anders ausgestaltet ist. Andere Proteine dagegen werden vorteilhaft in prokaryontischen Wirtszellen produziert, da hier kostengünstig große Mengen des Proteins einfach herstellbar sind. Weiterhin ist die Transfektion und gezielte Expression bestimmter Gene ein bedeutendes Werkzeug der Zell- und Molekularbiologie, um biologische Prozesse zu analysieren und zu charakterisieren. Die Transfektion von Pflanzenzellen ist eine wichtige Methode für Pflanzentechnologie bezüglich der Herstellung von Pflanzen mit neuen Eigenschaften und Inhaltsstoffen (transgene Pflanzen) oder herbizidresistenter Pflanzen. Schließlich werden im Rahmen von biomedizinischer Forschung auch regelmäßig Zellen mit einzelsträngigen Nukleinsäuren (ssDNA oder ssRNA) oder mit Nukleinsäureanaloga (beispielsweise Peptide Nucleic Acids, PNAs) transfiziert, die intrazellulär als Effektoren, beispielsweise als spezifische Inhibitoren von Proteinsynthesen durch sogenannte Antisense-Techniken, wirksam werden. In all diesen Prozessen und Methoden ist die Transfektion zur spezifischen Einbringung einer relevanten Nukleinsäuren ein wichtiger Verfahrensschritt.

In gleicher Weise sind auch Verfahren relevant, die direkt ein Protein oder Peptid in eine Zelle transportieren können: hier sind die wichtigsten Anwendungsfälle jeweils Therapeutika. Beispielsweise können intrazelluläre Antikörper dazu verwendet werden, spezifisch Pathogene in Zellen zu erkennen und zu inhibieren. Eine Methode hierfür ist die Elektroporation von Makromolekülen in die Zielzellen; diese Methode ist jedoch nur in vivo anwendbar und nur mit geringer Effizienz und relativ hohen Verlusten bezüglich der Zellen verbunden (vgl. E.J. Verspohl, I. Kaiserling-Buddemeier, A. Wienecke, *Introducing specific antibodies into e-lectropermeabilized cells is a valuable tool for eliminating specific cell functions*, Cell. Biochem. Funct. Band 15. S. 127-134. 1997).

Zum Transfer von Nukleinsäuren in Zellen sind nach dem Stand der Technik einige Methoden bekannt. Dazu zählen - wie bei dem vorstehend genannten Verfahren zum Transfer von Proteinen - physikalische Verfahren wie die Elektroporation, bei der durch Anlegen eines elektrischen Feldes die Membran von Zellen perforiert und damit durchlässig für große Makromoleküle wird. Elektroporation ist jedoch bei sensitiven Zellen oft schwierig und oft mit nur geringer Überlebensrate der Zellen verbunden. Im Falle von eukaryontischen Zellen werden nach dem Stand der Technik meist chemische Methoden verwendet, wie beispielsweise die Kopräzipitation von Calciumphosphat und DNA, oder die Bildung von höhermolekularen Komplexen, beispielsweise aus DEAE-(Diethylaminoethyl-)Dextran und DNA (vgl. Y.W. Yang & J.C. Yang, "Studies of DEAE-dextran-mediated gene transfer", Biotechnol. Appl. Biochem. 1997, Band 25. S. 47-51) oder von Dendrimeren mit DNA (vgl. J.F. Kukowska-Latallo, A.U. Bielinska, J. Johnson, R. Spindler, D.A Tomalia, & J.R. Baker Jr., "Efficient transfer of genetic material into mammalian cells using Starburst polyamidoamine dendrimers", Proc. Natl. Acad. Sci. U.S.A. 1996, Band 93, S. 4897-4902). Grundprinzip dieser Transfektionsreagenzien ist zunächst eine Kompaktierung des langen und mechanisch steifen Nukleinsäurefadens; diese Kompaktierung ist in den meisten Fällen eine wichtige Voraussetzung für die erfolgreiche Aufnahme der Nukleinsäuren in die Zellen. Gelegentlich wird dies auch durch ein Polykation erreicht wie beispielsweise reines oder modifiziertes Polylysin (vgl. hierzu beispielsweise Patent WO 98/06869).

Im medizinisch-pharmazeutischen Anwendungsbereich werden liposomenbasierte Systeme auf der Grundlage von kationischen Lipiden oft für Transfektionen bevorzugt; sie besitzen den Vorteil hoher Effizienzen. Dabei erfolgt ein Einschluß der Nukleinsäuren in kationische Liposomen verschiedener Herkunft und Zusammensetzung (vgl. die kommerziellen Produkte PerFect der Fa. Invitrogen, FuGene der Fa. Roche, Lipofectamine der Fa. Life Technologies, PolyFectin der Fa. Biontex, und LipoTaxi der Fa. Stratagene). Eine umfassende und aktuelle Übersicht dazu findet sich bei R.J. Lee & L. Huang, "Lipidic vector systems for gene transfer", Crit. Rev. Ther. Drug Carrier Syst. 1997, Band 14, S. 173-206. Hierzu existieren auch eine große Zahl von internationalen Patenten, die sich hauptsächlich durch die verwendete Formulierung der Liposomen unterscheiden.
Allerdings besitzen die genannten Techniken und Reagenzien jeweils verschiedene individuelle Nachteile. Zum einen sind die bestehenden Systeme (außer den liposomenbasierten Produkten) eher ineffizient, mit Ausbeuten von nur wenigen Prozent der zu transfizierenden Zellen; zum anderen sind gerade die effektiven (liposomenbasierten) Reagenzien zunehmend toxisch, insbesondere für empfindliche eukaryontische Zellen. Auch andere nachteilige Eigenschaften sind beschrieben; beispielsweise lösen sich bei der Transfektion von adhärenten eukaryontischen Zellen mit DEAE-Dextran die nur schwach angehefteten Zellen unerwünschterweise von ihrem Untergrund, der Zellkulturschale. Auch Transfektionsagenzien, die nicht unmittelbar toxisch für Zellen sind, können Eigenschaften aufweisen, die die Zellen nach der Transfektion beeinflussen. Weiterhin sind die existierenden Agenzien der neuen Generation wie beispielsweise Dendrimere (Produkt SuperFect - Fa. Qiagen) zwar in bestimmten Anwendungsfällen nur wenig toxisch, dafür jedoch aufwendig in der Herstellung und somit teuer. Schließlich erfordern viele der bisherigen Transfektionsprotokolle eine große Zahl von Manipulationen durch den Anwender und sind somit in ihrer Anwendung kompliziert und langwierig; auch kann dies die Reproduzierbarkeit von Transfektionsversuchen negativ beeinflussen. Die von Herstellern vorgegebenen Standardprotokolle für Transfektionen müssen für jeden Zelltyp individuell modifiziert und neu optimiert werden, um jeweils maximale Ausbeuten zu erreichen. Die Agentien sind nur für den Transport von doppelsträngiger DNA, nur selten für einzelsträngige Nukleinsäuren, und praktisch nie für andere molekulare Substanzen wie beispielsweise Proteine oder Peptide geeignet. Und nicht zuletzt bestehen die üblichen Transfektionsreagenzien nur aus jeweils einer oder wenigen molekularen Spezies und sind daher wenig variabel in ihrer Anwendbarkeit.

In US-A-5,965,404 sowie Fritz J. D. et al. "Gene transfer into mammalian cells using histone condensed plasmid DNA", Human Gene Therapy, Bd. 7, 1996, S. 1395-1404 und Boulikas T. et al., "Histones, protamine, and polylysine but not poly (E: K) enhance transfection efficiency", International Journal of Oncology, Bd. 10, Nr. 2, 1997, S. 317-322, ist die Verwendung eukaryontischer Histon- und Histonähnlicher Proteine für die Transfektion tierischer Zellen mit Nukleinsäuren beschrieben.

In der Veröffentlichung Esser D. et al., "The HU protein from Thermotoga maritima: Recombinant expression, purification and physicochemical characterization of an extremely hyperthermophilic DNA-binding protein", Journal of Molecular Biology, Bd. 291, Nr. 5, S. 1135-1146 ist beschrieben, dass TmHU auf nichtsequenzspezifische Weise DNA bindet.

Aufgabe der Erfindung ist es, die vorstehend genannten Nachteile nach dem Stand der Technik zu beseitigen oder abzumildern. Dazu wird erfindungsgemäß ein Verfahren nach Anspruch 1 zum Transfer von Nukleinsäuren und/oder Nukleinsäureanaloga, und/oder Nukleinsäuren und/oder Nukleinsäureanaloga und/oder Aminosäuren enthaltenden Substanzen, in prokaryontische und/oder eukaryontische Zellen bereitgestellt, wobei
- die zu transferierende Nukleinsäure oder das Nukleinsäureanalogon, oder die Nukleinsäuren oder Nukleinsäureanaloga oder Aminosäuren enthaltende Substanz mit einem prokaryontischen HU-Protein in Kontakt gebracht wird, um einen Komplex aus der Nukleinsäure, dem Nukleinsäureanalogon, oder der Nukleinsäuren oder Nukleinsäureanaloga oder Aminosäuren enthaltenden Substanz und dem prokaryontischen HU-Protein zu bilden,
und
- anschließend der Komplex aus Nukleinsäure, Nukleinsäureanalogon und/oder Nukleinsäuren oder Nukleinsäureanaloga oder Aminosäuren enthaltender Substanz und dem prokaryontischen HU-Protein mit den prokaryontischen oder eukaryontischen Zielzellen in Kontakt gebracht wird, um einen Transfer des Komplexes in die Zellen zu erreichen.

Vorteilhafte Ausführungsformen der vorliegenden Erfindung ergeben sich aus den Unteransprüchen und der Beschreibung.

### Beschreibung

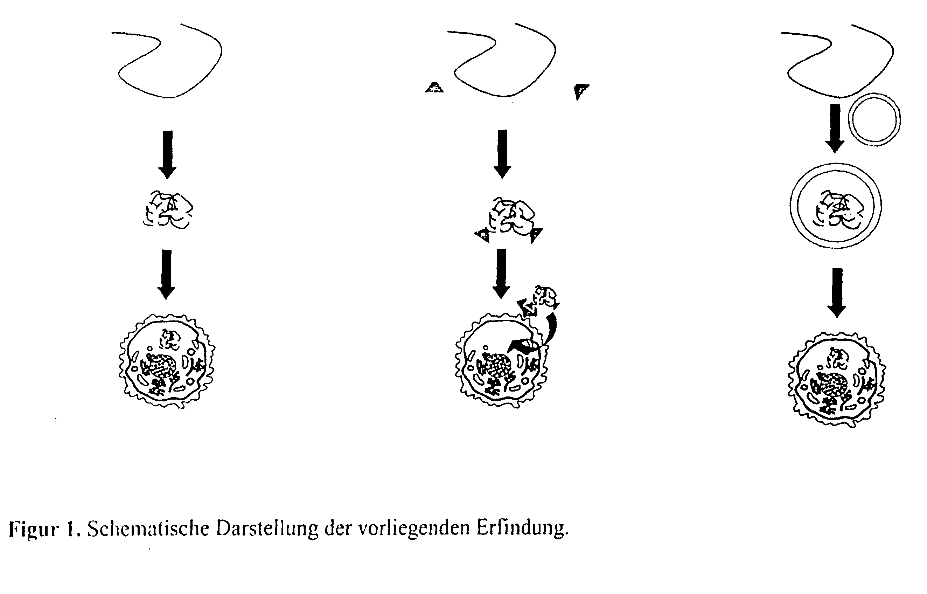

Die vorliegende Erfindung betrifft ein Verfahren zum Transfer von Nukleinsäuren, Nukleinsäureanaloga und/oder von Nukleinsäuren oder Nukleinsäureanaloga oder Aminosäuren enthaltenden Substanzen, insbesondere von Nukleinsäuren wie DNA in Form von Plasmiden, in prokaryontische oder eukaryontische Zellen.
Dabei wird ein prokaryontisches HU-Protein unter geeigneten Inkubationsbedingungen an die zu transferierende Substanz kovalent angebunden oder zur nichtkovalenten Assoziation mit der zu transfizierenden Nukleinsäure gebracht, und dieser Komplex zu den Zielzellen gegeben. Die besondere Eigenschaft des prokaryontischen HU-Proteins zur Kondensation von DNA begünstigt dabei wesentlich den Transfer von DNA-basierten Wirkstoffen. Zur Steuerung der Aufnahme und zur Erhöhung der Effizienz kann das prokaryontische HU-Protein weitere Ausgestaltungsmerkmale, beispielsweise in Form von zusätzlichen Fusionen, aufweisen. Die Zellen internalisieren den Komplex aus Protein und Wirkstoff bzw. Protein und Nukleinsäure. Verschiedene Ausführungsformen sind schematisch in Figur 1 gezeigt.

Zum Transfer wird ein prokaryontisches, Nukleinsäure-bindendes Protein, nämlich ein HU-Protein verwendet, vorzugsweise aus einem thermostabilen Organismus, weiterhin bevorzugt aus einem hyperthermophilen Organismus. Bei Verwendung von Nukleinsäuren als zu transferierende Substanz bildet das prokaryontische HU-Protein mit der Nukleinsäure einen reversiblen Komplex; das prokaryontische Protein kondensiert und kompaktiert die Nukleinsäure. Dieser Komplex ist dadurch auch vor unerwünschten Effekten, beispielsweise dem Abbau durch Nukleasen, geschützt. Solchermaßen kondensierte Nukleinsäuremoleküle können passiv oder aktiv über die Zellmembran von eukaryontischen Zellen oder die Zellwand von prokaryontischen Zellen nach entsprechender Inkubation in diese Zellen aufgenommen werden. Wird als Nukleinsäure dabei ein kodierendes Gen in Form einer entsprechenden DNA (beispielsweise als Plasmid) verwendet, dann kann das Gen anschließend von der Zielzelle exprimiert werden. Wird als zu transportierende molekulare Substanz dagegen ein Protein oder ein Peptid verwendet, dann ist eine Fusion des zu transferierenden Proteins oder Peptids mit dem Transferprotein auf gentechnischer Ebene günstig. Zur Erhöhung der Effizienz kann das HU-Protein mit Effektoren auf der Basis von Proteinen oder Peptiden verknüpft (fusioniert) werden. Weiterhin kann die Verwendung eines Proteins vorteilhaft sein, welches eine typische Signatur für Kerntranslokation in seiner Sequenz aufweist.

Weiterhin kann eine Umhüllung des Komplexes mit Lipiden, Polyethylenglykol, oder anderen Molekülen vorteilhaft bezüglich der Erhöhung der Effizienz und bezüglich der biologischen Eigenschaften des Komplexes sein.

Ein wichtiges Merkmal bei der Transfektion von Nukleinsäuren ist deren Kondensation. Diese Moleküle besitzen üblicherweise in Lösung eine lange, unflexible Struktur, die nur wenig biegsam ist. Aufgrund der umfangreichen kodierenden Länge von Genen kann die DNA sehr lang sein und damit für die Transfektion ungünstige physikalische Eigenschaften aufweisen. Kondensation ist in dieser Erfindung die Verminderung des ungünstigen Längen-zu-Durchmesser-Verhältnisses der DNA, durch Zusammenballung des DNA-Fadens oder auch eines zirkulären Plasmides zu einer kompakten Struktur, also auf ein kleines Volumen. Optimale Kondensation zeichnet sich durch die Entstehung einer nahezu globulären Struktur aus. Kondensation von Nukleinsäuren setzt das Vorhandensein von Kondensationsagentien voraus; eine übliche Funktion dieser Kondensationsagentien ist dabei die Kompensation der negativen Ladungen auf dem Polyphosphat-Rückgrat der DNA und anderer Nukleinsäuren.

Nach Maßgabe der Erfindung stammt das prokaryontische HU-Protein vorteilhafterweise aus einem hyperthermophilen Organismus, also einem bei Temperaturen von über 75 °C existierenden Lebewesen. Der Vorteil von Proteinen aus diesen Organismen besteht unter anderem darin, daß sie aufgrund ihrer besonderen Stabilitätseigenschaften sehr einfach handhabbar sind und beispielsweise eine Kühlung der Agenzien bei der Aufreinigung und Lagerung nicht notwendig ist. Weiterhin lassen sich Proteine aus diesen Organismen einfach und in großer Ausbeute rekombinant in *Escherichia coli* herstellen und reinigen. Dabei kann die hohe Stabilität der Proteine gegen Denaturierung dazu genutzt werden, eine Reinigung unter sehr stringenten Bedingungen durchzuführen und damit mögliche Kontaminationen in den Präparationen, auch beispielsweise durch bakterielle Lipopolysaccharide (Endotoxine), DNAsen, Proteinasen, RNAsen, u.a. auf ein Minimum unterhalb der Nachweisgrenze gängiger analytischer Methoden zu begrenzen. Hilfreich ist hier auch die Eigenschaft des in dieser Erfindung vorzugsweise verwendeten Proteins TmHU *(Thermotoga maritima* HU-Protein), spektroskopisch im Wellenlängenbereich von 260 bis 300 nm transparent zu sein; dadurch ist eine schnelle und empfindliche spektroskopische Analytik von Verunreinigungen durch Nukleinsäuren oder andere Proteine einfach möglich. Bevorzugt für reproduzierbare und effiziente Transferraten von Nukleinsäuren, Nukleinsäureanaloga und/oder Aminosäuren enthaltender Substanzen sind erfindungsgemäß reine Präparationen der Transferagenzien, da Kontaminationen, insbesondere von bakteriellen Endotoxinen, aber auch von Proteinasen, DNasen und RNasen, die Transferergebnisse verschlechtern können. Dies gilt insbesondere für sensitive Zellen beziehungsweise für Verfahren mit geringer Transfereffizienz.

Histone sind Proteine in eukaryontischen Zellkernen, die Nukleinsäuren vorwiegend oder gänzlich unspezifisch binden und deren Zweck hauptsächlich in der Kompaktierung (Kondensation) der DNA liegt, beispielsweise durch Ladungsneutralisation und hydrophobe Effekte, vermittelt durch das Protein. Somit verringern sie den effektiven Raumanspruch der DNA innerhalb des Zellkerns. HU-Proteine bzw. histonähnliche Proteine erfüllen nach derzeitigem Kenntnisstand eine analoge Aufgabe in den einfacher strukturierten (kernlosen) prokaryontischen Organismen. Beispielsweise ist das DNA-bindende Protein aus dem hyperthermophilen Organismus *Thermotoga maritima* (histonähnliches Protein) den aus Eukaryonten stammenden Histonen nicht nur in der Stabilität überlegen, sondern auch in der Handhabbarkeit und bezüglich der geringeren strukturellen Komplexität. So sind beispielsweise eukaryontische Histone Assoziate von bis zu acht verschiedenen Proteinuntereinheiten, die alle separat hergestellt und in einem Komplex mit DNA assembliert werden müßten; dies ist *in vitro* kompliziert und aufwendig. In der vorliegenden Erfindung werden prokaryontische, HU-Proteine, bevorzugt das aus dem hyperthermophilen Organismus *Thermotoga maritima* stammende HU-Protein, verwendet.

Die von den Erfindern durchgeführten Versuche zeigen überraschend, daß dieses prokaryontische DNA-bindende, histonähnliche Protein HU aus dem hyperthermophilen Eubakterium *Thermotoga maritima* (Gattung Thermotogales), nachfolgend benannt TmHU, nicht nur Nukleinsäuren bindet, schützt und kondensiert, sondern darüber hinaus auch in der Lage ist, diese Nukleinsäuren außergewöhnlich effektiv in verschiedene Zelltypen zu transportieren. Dabei sind die Effizienzen einer nachfolgenden Genexpression signifikant höher als die vergleichbarer, etablierter Agenzien, ohne daß dabei Anzeichen von zellulärer Toxizität erkennbar sind. Das Transferprotokoll ist einfach und robust und erfordert einen sehr geringen zeitlichen Aufwand für die Durchführung des Transfers.

In gleicher Weise konnte gezeigt werden, daß auch Aminosäuren enthaltende Substanzen, beispielsweise Proteine oder Peptide, bevorzugt Proteine, die an TmHU fusioniert werden, vermittels der Aufnahmewirkung von TmHU in die Zielzellen aufgenommen werden.

Als vorteilhaft hat sich dabei weiter der Umstand erwiesen, daß sich das HU-Protein in sehr hoher Ausbeute rekombinant im Bakterium *Escherichia coli* herstellen und einfach sowie kostengünstig in spektroskopisch reiner Form isolieren läßt. Der hyperthermophile Ursprung erfordert beispielsweise keine Aufarbeitung und Lagerung des Proteins unter gekühlten Umgebungsbedingungen. Darüber hinaus geschieht der primäre Schritt der Reinigung, die Trennung des HU-Proteins von der Masse der *Escherichia coli-*Wirtsproteine, durch eine einfache Hitzefällung, bei der praktisch ausschließlich das thermostabile TmHU-Protein in Lösung bleibt.

Es konnte gezeigt werden, daß das TmHU-Protein hervorragende Eigenschaften bezüglich der Transfektion von Zellen aufweist. Das Protein kompaktiert (kondensiert) Nukleinsäuren zu einem stabilen Komplex. Dadurch ist ein ausgezeichneter Schutz vor Abbau der Nukleinsäuren durch Nukleasen gegeben. Die Komplexe werden effizient in Zellen aufgenommen.

Weiterhin können zusätzlich Peptide oder Proteindomänen an das TmHU-Protein gentechnisch fusioniert werden, die den Komplexen veränderte Eigenschaften geben. So können modifizierte TmHU-Proteine eine Erhöhung der Aufnahmeeffizienz oder eine gerichtete Aufnahme in die Zellen erlauben. Als Modifikationen kommen beispielsweise Peptide zur Bindung an zelluläre Oberflächenstrukturen in Frage; so ist bekannt, daß Peptide mit dem Sequenzmotiv Arginin - Glycin - Aspartat (RGD) bevorzugt an häufig vorhandene Integrine vom Typ αᵥβ₃ oder αᵥβ₅ auf der Zelloberfläche binden. Von anderen Peptidmotiven ist bekannt, daß sie durch ihre amphipathische strukturelle Natur eine Insertion in eukaryontische Zellmembranen bewirken und dadurch eine Aufnahme von Molekülen durch die Zellen bewerkstelligt werden kann. Schließlich kann die Erhöhung der Aufnahmeeffizienz und eine Spezifizierung von Zelltypen auch durch die Verwendung von Proteinen oder Proteindomänen erreicht werden, die als Effektoren an zelluläre Oberflächenrezeptoren binden. Ein Beispiel hierfür ist der Epidermal Growth Factor EGF, der an den (auf einigen Tumorzelltypen überexprimierten) EGF-Rezeptor hoch spezifisch binden kann. Dadurch kann die Aufnahme der TmHU/EGF-DNA-Komplexe spezifisch in solche Tumorzellen erfolgen. Aber auch nach der Aufnahme der Komplexe in Zellen können anhaftende Funktionsdomänen zur Steigerung der Effizienz beitragen. Hier sind als Beispiele Proteine zur Bewerkstelligung einer Endosomen-Freisetzung, und Peptide zur gezielten Aufnahme in eukaryontische Zellkerne durch NLS-Sequenzen (NLS, nuclear localization signal) zu benennen.

Eine Ummantelung der Protein/Nukleinsäure-Komplexe zur weiteren Abgrenzung von der Umgebung sowie gegebenenfalls zur Erhöhung der Transfektionseffizienz kann durch Umhüllung mit einer Liposomen-Membran bewerkstelligt werden. Liposomen können nach dem Stand der Technik einfach hergestellt werden; dabei erfolgt ein passiver Einschluß der Protein/Nukleinsäure-Komplexe in die Liposomen. Diese Konstrukte haben den Vorteil verminderter Immunogenität bei einer *in vivo*-Anwendung in Organismen. Darüber hinaus kann durch die Verschmelzung der Liposomenhülle mit der Zellmembran eine Erhöhung der Zellaufnahme erfolgen. Eine Ummantelung der Protein/Nukleinsäure-Komplexe kann auch durch die Verwendung anderer molekularer Substanzen erfolgen, beispielsweise durch Polyethylenglycol der Molekularmasse 2000 Da (PEG 2000). Nach dem Stand der Technik wurde bereits gezeigt, daß eine derartige PEGinylierung zu einer erheblichen Verminderung der Immunantwort bei *in vivo*-Anwenduneen führt (vgl. beispielsweise die Zusammenfassung bei M.D. Scott & K.L. Murad, "Cellular camouflage: fooling the immune system with polymers", *Curr. Pharm. Dcs.* 1999, Band 4, S. 423-438).

Der Transfer von Nukleinsäuren, Nukleinsäureanaloga und/oder Aminosäuren enthaltender Substanzen in Zellen ist in den nachstehenden Beispielen jeweils beispielhaft am Transfer von kodierenden DNA-Sequenzen zur Transfektion eines Reportergenes, sowie am Transfer eines Proteins (GFP) in Zielzellen gezeigt. Als prokaryontisches Protein wird dabei das HU-Protein aus *Thermotoga maritima* verwendet, das sich als sehr gut geeignet für das beschriebene Verfahren erwiesen hat. Das Protein besitzt zwei typische Signaturen für eine Kerntranslokation bei Transfer des Proteins in eukaryontische Zellen, vergleichbar mit der Translokationssequenz RPAATKKAGQAKKK nach Robbins, Dilworth, Laskey, und Dingwall, *Cell* 64, S. 615-623, 1991; damit ist eine Kerntranslokation des Proteins sowie der mit dem Protein assoziierten molekularen Substanzen (DNA, Proteine) mit einer Wahrscheinlichkeit von über 95 % gegeben (ermittelt mit dem nach dem Stand der Technik verwendeten Computerprogramm PSORT II). Diese Kerntranslokation ist besonders beim Transfer von DNA in Zellen von hoher Bedeutung für die große Effizienz des Verfahrens. Das nachfolgende Beispiel 8 zeigt aber deutlich, daß das Protein nicht nur DNA in Zellen transferieren kann, sondern auch Proteine, die an TmHU angehängt sind. Damit besitzt das Protein auch die vorteilhafte Eigenschaft, eukaryontische Zellmembranen zu passieren. Durch das Anhängen von Rezeptorbindungsmodulen wie beispielsweise EGF (vgl. nachstehendes Beispiel 6) kann dabei auch eine gerichtete Aufnahme in Zellen erreicht werden.

Ein besonderer Vorteil des TmHU-Proteins ist seine fehlende Toxizität in den nachstehend genannten Beispielen, sowie der Umstand, daß adhärente Zellen bei der Transfektion nicht von den Zellkulturgefäßen abgelöst werden. Diese Eigenschaften setzen das Protein von anderen Transfektionsagentien wie Liposomen oder synthetischen Stoffen (Dendrimere) ab Gleichfalls ist die Eigenschaft von TmHU, aus einem hyperthermophilen Organismus zu stammen, für die Anwendung des Systems von besonderem Vorteil. So ist die rekombinante Herstellung besonders einfach, und es kann eine hohe Reinheit der Proteinpräparation gewährleistet werden. Die extrem hohe Thermostabilität des Proteins erlaubt lange und stabile Lagerung auch unter ansonsten ungünstigen Umweltbedingungen. Insbesondere aber ist es möglich, die Protein/Nukleinsäurekomplexe, die für die Transfektion notwendig sind, bei hohen Temperaturen zu generieren. Wird DNA mit dem TmHU-Protein in geeigneten Mengenverhältnissen vermischt und kurzzeitig für etwa 0.5 bis 10 min auf hohe Temperaturen (um etwa 90 °C) gebracht, dann wird ein feines Präzipitat aus DNA und TmHU gebildet, das für besonders hohe Transfektionseffizienzen geeignet ist. Eine solche Möglichkeit steht nur zur Verfügung, wenn hyperthermophile Nukleinsäure-bindende Proteine eingesetzt werden. Die Inkubation der Nukleinsäuren mit dem TmHU-Protein bei üblichen Raumtemperaturbedingungen kann dabei zwischen 0.5 und 180 min betragen, wobei hier eine Inkubation von 60 min in der Regel ausreichend ist. Für den Transfer von Nukleinsäuren mit TmHU hat es sich als vorteilhaft erwiesen, dem TmHU/DNA-Gemisch bei der Herstellung der Komplexe zusätzlich Calcium zuzusetzen, das die Komplexbildung beschleunigt. Die Inkubationszeit der bei Raumtemperatur oder bei hoher Temperatur gebildeten Präzipitate mit den Zielzellen kann je nach Zelltyp und Anwendungsfall zwischen 30 Sekunden und mehreren Tagen betragen.

Besonders gut geeignet sind für die vorliegende Erfindung HU-Proteine und davon abgeleitete Proteine aus thermophilen und insbesondere hyperthermophilen prokaryontischen Organismen. Hier kommen aus dem Reich der Archaea insbesondere Vertreter der Crenarchaeota (hyperthermophile Archaebakterien) in Frage, mit Vertretern der taxonomischen Gruppen der Pyrodictiales, Sulfolobales, Thermoproteales, oder die unklassifizierten Crenarchaeota wie Aeropyrum, Caldococcus, Cenarchaeum, Igneococcus, Pyrolobus, Sulfophobococcus, Thermodiscus, und Thermosphaera. Bei den Euryarchaeota sind insbesondere Proteine aus den taxonomischen Klassen der Thermococcales und der Thermoplasmales relevant. Aber auch thermophile und hyperthermophile Eubakterien besitzen natürlicherweise HU-Proteine, die im Rahmen einer erfindungsgemäßen Anwendung eingesetzt werden können; dazu gehören beispielsweise die Vertreter der taxonomischen Klassen der Thermosulfobakterien, die Aquificales, Thermotogales, oder die Gruppe der Thermus/Deinococcus-Bakterien.

Neben Proteinen aus hyperthermophilen oder thermophilen Organismen können auch Proteine aus anderen prokaryontischen Organismen für eine erfolgreiche Transfektion eingesetzt werden, wie dies das nachstehende Beispiel 8 (unter Verwendung des HU-Proteins aus *Escherichia coli)* demonstriert. Hier erfolgt die effiziente Bildung der zur Transfektion mit Nukleinsäuren notwendigen Komplexe mit dem prokaryontischen Protein vorzugsweise durch Zugabe geeigneter Mengen an Calcium zum Gemisch; auf einen Hitzeschritt zur Herstellung der feinen Präzipitate muß hier verzichtet werden. Die Proteine können dabei aus mesophilen Organismen der Reiche Archaebakterien oder Eubakterien isoliert werden, oder Modifikationen dieser natürlichen Proteine sein. Als Vertreter der Archaebakterien sind hier die taxonomischen Gruppen der Korarchaeota und der Euryarchaeota zu nennen, letztere mit den Familen der Archaeoglobales, Halobacteriales, Methanobacteriales, Methanococcales, Methanomicrobiales, Methanopyrales, Methanosarcinales, Thermococcales, und Thermoplasmales. Zu den taxonomischen Gruppen der Eubakterien, die überwiegend nichtthermophile oder nicht-hyperthermophile Organismen umfassen, gehören in diesem Zusammenhang insbesondere die Aquificales, die Gruppe der Chlamydiales/Verrucomicrobia, die Gruppe der Coprothermobacter, die Cyanobacteria, die Gruppe der Cytophagales/Grüne Schwefelbakterien, die Gruppe der Fibrobacter/Acidobacteria, die Firmicutes, die Gruppe der Flexistipesk, die Fusobacteria, die Gruppe der Holophaga, die Gruppe der Nitrospira, die Planctomycetales, die Proteobacteria, die Spirochaetales, und die Gruppe der Synergistes.

Bei der Verwendung von prokaryontischen Organismen als Zielzellen für die beschriebene Transfertechnik kann die Bildung der feinen Präzipitate durch die kurzzeitige Erhitzung des TmHU/DNA-Gemisches für eine Steigerung der Transfektionseffizienz sorgen, sofern ein Protein aus einem thermophilen oder einem hyperthermophilen Organismus verwendet wird. Neben einer Transfektion, die durch einfache Inkubation der TmHU/DNA-Komplexe mit den prokaryontischen Zellen erfolgt, kann auch eine Elektroporation nach Stand der Technik mit Hilfe der TmHU/DNA-Komplexe erfolgreich sein. Die DNA wird dabei durch das TmHU geschützt und in seiner Struktur stabilisiert. In nachfolgendem Beispiel 1 konnte gezeigt werden, daß TmHU auch in hohen Konzentrationen für *Escherichia coli* nicht toxisch ist. In analoger Weise kann auch ein Transfer von Proteinen oder anderen molekularen Substanzen in prokaryontische Zellen erfolgreich durchgeführt werden.

Gleichfalls schützt das TmHU-Protein *in vivo* Nukleinsäuren vor enzymatischem Abbau. Das rekombinant hergestellte Protein bindet in den Bakterienzellen an die dort vorhandenen Nukleinsäuren wie beispielsweise Plasmide. Auf dieser Basis läßt sich ein System herstellen, das unter wirtschaftlichen Bedingungen zur Produktion von großen Mengen von Nukleinsäuren in hoher Reinheit und ohne der Gefahr des enzymatischen Abbaus durch Nukleasen geeignet ist.

Ein besonders wichtiges Anwendungsgebiet für den Transfer von molekularen Substanzen in Zellen ist auch die Verwendung von Pflanzenzellen als Zielzellen. Diese Pflanzenzellen zeichnen sich durch eine stabile, kaum durchdringliche Zellwand aus, daher sind derzeit kaum Methoden bekannt, um molekulare Substanzen wie DNA oder Proteine in Pflanzenzellen zu transportieren. Die außerordentlich stabilen TmHU/DNA-Protein-Komplexe können jedoch dazu geeignet sein, unter üblichen Verfahren nach dem Stand der Technik wie chemische Transfektion, Elektroporation, oder auch durch einfache Inkubation der TmHU/DNA-Komplexe einen Transfer in die Pflanzenzellen zu erreichen. Dabei spielen wiederum die im TmHU vorhandenen Kerntranslokationssequenzen eine wichtige Rolle bezüglich der erreichbaren hohen Effizienz des in der vorliegenden Erfindung beschriebenen Verfahrens.

Die gentechnische Veränderung von Pflanzen kann beispielsweise durch die Züchtung ertragreicherer Sorten mit günstigeren Eigenschaften einen wesentlichen Beitrag zur Sicherung der Weltemährung leisten. In Westeuropa sind gut 20 % an Verlusten durch Krankheiten, Schädlinge und Unkräuter zu verzeichnen. Eine wichtige Anwendung der Erfindung kann somit auch in der Transfektion von Pflanzenzellen liegen. Zur Transfektion von Pflanzenzellen sind jedoch bisher nur wenige, teilweise sehr aufwendige, unzureichende oder teure Verfahren des Gentransfers bekannt. Die populärste Methode ist dabei die Verwendung des am Wurzelhals Tumore verursachenden Bakteriums *Agrobacterium tumefaciens,* das die DNA in die Zellen einbringt. Das Bakterium befällt jedoch nur zweikeimblättrige Pflanzen; einkeimblättrige Pflanzen (darunter wichtige Getreidesorten) werden hingegen nicht transfiziert. Weitere, bisher nur unzureichend wirksame und vor allem aufwendige Möglichkeiten zum Gentransfer sind Elektroporation und Mikroinjektion. Des weiteren wird die Methode des Partikelbeschusses (..magic bullets") verwendet, die oft erfolgreich, jedoch auch teuer ist. Die Verwendung des Komplexes aus TmHU und einer bestimmten molekularen Substanz kann diesen Transfektionsmethoden in Effizienz und Kosten erheblich überlegen sein. Dabei kommen vor allem auch Modifikationen des Proteins zum Einsatz, die beispielsweise die ansonsten undurchlässige Zellwand von Pflanzenzellen durch an TmHU anfusionierte Enzyme lokal begrenzt abbauen können und somit einen geeigneten Aufnahmeweg für den TmHU/DNA-Komplex bereitstellen. Eine Anwendung dafür liegt vor allem auf dem Gebiet der Ertragssteigerung durch die Entwicklung ertragreicherer bzw. ernährunesphysiologisch wenvollerer Sorten (Veränderung oder Steigerung des Gehaltes von Inhaltsstoffen wie Proteinen, Fettsäuren, Geschmacksstoffen oder Stärke), durch die Vermeidung von Schädlingsbefall (Einbringen von Resistenzgenen, deren Genprodukt für den jeweiligen Schädling toxisch ist), durch die Vermeidung von Pflanzenkrankheiten sowie die Verhinderung von Unkrautwachstum (durch Herbizidresistenzgene) und das Einbringen von Fremd-DNA zur Produktion von Wirkstoffen in den Pflanzen. Dies kann durch eine erfindungsgemäße Anwendung des prokaryontischen Nukleinsäure-bindenden Proteins erfolgen, mit dem Plasmid-DNA in Pflanzen eingebracht werden kann.

Als zu transportierende molekulare (therapeutische) Substanz bei medizinischer Anwendung kann beispielsweise DNA verwendet werden, die für intrazellulär oder extrazellulär wirkende Proteine codiert. Die therapeutische DNA kann dabei einzel- oder doppelsträngig in die Zelle eingeführt werden. Ebenfalls wäre eine Kopplung sequenzspezifischer Oligonukleotide an die prokaryontischen Proteine denkbar; die durch Hybridisierung mit der therapeutischen ssDNA eine spezifische Komplexbildung mit dem prokaryontischen Protein vermitteln; dies würde die vorstehend beschriebene unspezifische Nukleinsäurebindung durch eine spezifische Bindung substituieren. Ein weiterer Ansatzpunkt für den Transfer von therapeutischen Wirkstoffen wäre die Kömplexbildung mit Ribozymen, die eine spezifische Erkennungssequenz für eine RNA besitzen, die mit pathologischen Zuständen assoziiert ist. Diese RNA wird durch die Bindung der Ribozyme katalytisch gespalten und inaktiviert.

Eine Therapie kann alternativ zu Nukleinsäuren auch durch in die Zelle eingebrachte Proteine oder Peptide erfolgen. Beispielsweise könnte eine HIV-Therapie auf erfindungsgemäß eingebrachten trans-dominanten (modifizierten) Proteinen basieren, die dann mit nativen HIV-Proteinen in der Zelle konkurrieren und somit ihre Funktion inhibieren. Ebenfalls können Peptide oder synthetisch modifizierte Peptide die Wirkung bestimmter HIV-Proteine, beispielsweise der HIV-Protease, inhibieren. Außerdem können Proteine oder Peptide direkt an das prokaryontische Protein fusioniert werden, in der Weise, daß sich zwischen therapeutisch wirksamer Substanz und prokaryontischem Transportprotein eine Erkennungssequenz für HIV-Protease oder eine zelluläre Protease befindet, die das Protein oder Peptid intrazellulär (und ggf. wiederum spezifisch in infizierten Zellen) freisetzt. Dies würde die Festlegung der Spezifität einer (therapeutischen) Wirkung auf bestimmte Zelltypen ermöglichen, wobei das verwendete prokaryontische Protein lediglich das Transportvehikel für die Passage in die Zellen darstellt.

Eine weitere Anwendung der vorliegenden Erfindung ist die Applikation von Anti-Tumor-Wirkstoffen bei malignen Erkrankungen. Dafür müssen die hergestellten Komplexe Bausteine enthalten, die den Transport des Wirkstoffes in Tumorgewebe gewährleisten. Je nach Art des Tumors geschieht dies beispielsweise durch in die Wirkstoff/Protein-Komplexe eingebaute Antikörper, die an Tumorantigene binden, die ausschließlich oder weitestgehend nur auf Tumorzellen vorhanden sind. Solide Tumore benötigen eine ausreichende Blutversorgung und sekretieren deshalb Wachstumsfaktoren, die die Bildung neuer Blutgefäße im Tumorgewebe initiieren. Die Epithelzellen neu gebildeter Blutgefäße exprimieren verstärkt plasmamembrangebundene Integrine. Diese Rezeptoren erkennen spezifisch die Sequenzabfolge RGD (Arginin-Glycin-Aspartat) und bewirken eine rezeptorvermittelte Endocytose RGD-haltiger Liganden. Diese Eigenschaft kann ebenfalls zur Adressierung von Tumorzellen und damit verbundenem epithelialen Gewebe genutzt werden, indem RGD-exponierende Peptide an die prokaryontischen Transportproteine anfusioniert werden, so daß eine Aufnahme der therapeutischen Substanz in das Tumorgewebe erfolgt. Andere Tumore zeigen eine signifikant erhöhte Präsentation des natürlichen EGF-Rezeptors auf der Zelloberfläche. In diesem Falle bietet es sich an, eine Spezifität der Aufnahme der in der vorliegenden Erfindung beschriebenen Komplexe durch Anfusionieren der EGF-Domäne an das prokaryontische Transportprotein zu bewerkstelligen, wie dies in nachfolgendem Beispiel 6 beschrieben ist. Eine Kombination verschiedener Rezeptorbindungseigenschaften bewirkt neben einer verbesserten Gewebespezifität eine Therapie, die gleichzeitig an mehreren Stellen den Tumors angreift und die Bildung wirkstoffresistenter Zellen verringert. Als Wirkstoffe können hier Nukleinsäuren, wie einzel- und doppelsträngige DNA oder RNA eingesetzt werden. Die darauf codierten Proteine können beispielsweise in der Zelle Apoptose auslösen, indem sie an den entsprechenden Stellen in den zellulären Signaltransduktionskaskaden eingreifen. Zu einer erweiterten Tumorspezifität und somit höheren Sicherheit lassen sich zur Transkription Promotoren verwenden, die bevorzugt in Tumorzellen aktiv sind. In gleicher Weise können Peptide als zu transportierende molekulare Substanz eingesetzt werden, die eine Inhibition von Matrix-Metalloproteinasen bewirken. Insbesondere die Inhibition von MMP-2 und MMP-9 durch spezifische, kurze Peptidsequenzen kann hier eine effektive Wirkung zeigen.

Grundsätzlich läßt sich die hier beschriebene Erfindung auch zur Korrektur angeborener genetischer Defekte anwenden, wie beispielsweise ADA-Defizienz, Hämophilie, Duchenne-Muskeldystrophie, und Cystische Fibrose. Diese Erkrankungen sind monokausal, das heißt, sie lassen sich auf den Defekt eines einzelnen Gens zurückführen. Das Einbringen dieses Gens in korrekter Form ist damit in der Regel ausreichend, um die Krankheitssymptome aufzuheben oder zu vermindern. Für diese Anwendungen muß eine stabile Genexpression erreicht werden, entweder durch stabile episomale Vektoren oder eine Integration der therapeutischen DNA in zelluläre Chromosomen. Dafür können die übertragenen Nukleinsäuren Sequenzen enthalten, die eine Intgration erleichtern. Beispielsweise kann einzelsträngige DNA verwendet werden, die an ihren Enden ITR-Sequenzen (Inverted Terminal Repeats) des Adenoassoziierten Virus trägt, die zur chromosomalen Integration beitragen. Außerdem können neben der therapeutischen DNA oder RNA Proteine mit in die Zelle transportiert werden, die eine Integration aktiv katalysieren, wie beispielsweise HIV-Integrase, oder Rep78 und Rep68 des Adenoassoziierten Virus.

Die Expression korrigierender Gene kann idealerweise unter Kontrolle der natürlichen Promotoren erfolgen, wodurch gleichzeitig eine angepaßte Regulation gewährleistet wird. Ein zelltypspezifisches Targeting des Komplexes aus DNA und nukleinsäurebindendem prokaryontischen Protein ist daher in vielen Fällen nicht notwendig. Beispielsweise können bei Hämophilie-Patienten die fehlenden Faktoren der Blutgerinnungskaskade in Muskelgewebe produziert werden, wobei die Faktoren mit einer geeigneten Signalsequenz fusioniert werden, so daß sie aus der Zelle sekretiert werden und an ihren Wirkungsort, die Blutbahn, gelangen.

Außer den in verschiedenen, vorstehend diskutierten Beispielen genannten Nukleinsäuren können auch Proteine oder Peptide transferiert werden, die Apoptose oder Nekrose auslösen. Geeignet sind beispielsweise katalytische Domänen bakterieller Toxine (beispielsweise Diphtheria-Toxin, Cholera-Toxin, Botulinus-Toxin, und andere), die mit hoher Effizienz die Proteinbiosynthese der Zelle hemmen und dadurch Nekrose auslösen. Vorteilhaft kann sich dabei auswirken, daß nur wenige Moleküle nötig sind, um eine Zelle abzutöten. Ein weiterer therapeutischer Ansatz stellt der Transport der Thymidinkinase von Herpes-Simplex-Virus in Tumorzellen dar. Dieses Enzym phosphoryliert Nukleotidbausteine und weist dabei eine verringerte Substratspezifität gegenüber den zellulären Kinasen auf, so daß auch künstliche Nukleotide, wie beispielsweise Ganciclovir, phosphoryliert werden. Phosphoryliertes Ganciclovir wird bei der DNA-Replikation in neusynthetisierte DNA-Stränge mit eingebaut und führt zum Abbruch der Replikation, was wiederum die Zellteilung verhindert.

In vielen Fällen einer praktischen Anwendung ist eine effiziente Freisetzung der transportierten Substanzen innerhalb der Zelle notwendig, das heißt, die Substanz muß die endosomale Membran erfolgreich passieren. In den nachstehend genannten Beispielen ist diese Endosomenfreisetzung nicht limitierend, da eine Genexpression oder der Transport eines Proteins (GFP als Markerprotein, vgl. Beispiel 7) in der Regel mit hoher Effizienz erfolgt. Sollte eine Limitierung durch Endosomeneinschluß der aufgenommenen Komplexe erfolgen, dann kann diese Funktion durch Hämolysine, insbesondere thiolaktivierte Cytolysine, Translokationsdomänen bakterieller Toxine, oder bestimmte virale Proteine, wie beispielsweise das Adenovirus-Pentonprotein, realisiert werden, die in den zu transferierenden Komplex mit eingebracht werden. Des weiteren kann diese Funktion auch von chemischen Substanzen, wie beispielsweise Polykationen oder Dendrimeren, übernommen werden, die an die Komplexe mit angebunden werden.

Oft ist es für Anwendungen *in vivo* notwendig, die Immunogenität des aufgenommenen Komplexes so gering wie möglich zu halten. Die humorale Immunogenität des Komplexes selbst und die Erkennung und Eliminierung durch Makrophagen kann durch eine Maskierung mit Polyethylenglycol oder eine Umhüllung mit einer Lipiddoppelschicht erreicht werden, wie dies in nachfolgendem Beispiel 9 gezeigt ist. Polyethylenglycol kann beispielsweise chemisch so modifiziert werden, daß es kovalent an spezifische SH-Gruppen gebunden wird. Die immunogenität des therapeutischen Wirkstoffes, das heißt, der direkt eingeführten Proteine oder der von therapeutischen Nukleinsäuren transkribierten und/oder translatierten Proteine, kann mit einer Fusion von 35 bis 40 GA- (Glycin-Alanin)-repetitiven Sequenzen reduziert werden. GA-reiche Sequenzen kommen natürlicherweise bei dem EBNA1-Protein des humanen Eppstein-Barr-Virus vor und schützen das virale Protein vor einem Abbau durch das zelluläre Proteasom und einer Präsentation auf MHC-Klasse-1-Rezeptoren. Diese Schutzfunktion kann für die verschiedenen im Rahmen der vorliegenden Erfindung verwendeten Proteine und Peptide ausgeführt sein.

Neben Nukleinsäuren, Proteinen und Peptiden können für das in der vorliegenden Erfindung beschriebene Verfahren zum Transfer von molekularen Substanzen in Zellen auch andere Molekülklassen verwendet werden. So können Peptidderivate, Peptidantibiotika, Proteine mit modifizierten Seitenketten wie Fluoreszenzmarkierungen, Alkylierungen, Acetylierung, Peptid- oder Proteinkonjugate mit Kohlenhydrat-, Nukleinsäure- oder Lipidanteilen und analogen Veränderungen in gleicher Weise in den Komplex mit eingebaut werden. Ebenfalls lassen sich außer den üblicherweise verwendeten kodierenden (doppelsträngigen) Plasmiden auch einzelsträngige DNA, einzelsträngige oder doppelsträngige RNA, chromosomale DNA oder Chromosomenfragmente, Antisense-RNA, Ribozyme, katalytische RNA, Nukleotide, synthetische Nukleinsäuren wie beispielsweise Peptide Nucleic Acids (PNA), oder Hybride hiervon mit dem prokaryontischen Transportprotein koppeln, durch Interaktion mit der nukleinsäurebindenden Stelle des Proteins oder auch auf chemischem Wege. Sie sind dann dazu geeignet, mit hoher Effizienz in vorgegebene Zellen aufgenommen zu werden. Als zu transportierende nicht-nukleinsäureartigen Substanzen kommen im Rahmen der erfindungsgemäßgen Anwendung insbesondere Proteine wie beispielsweise Antikörper, antikörperanaloge Substanzen, Proteindomänen, Glykoproteine, Enzyme, Peptide, Peptidhormone, pharmazeutische Wirkstoffe auf Aminosäurebasis, Lipoproteine sowie Strukturproteine in Betracht.

Die Bindung der zu transportierenden Substanz an das prokaryontische Transportprotein kann unter dem Aspekt verschiedener physikalischer Wechselwirkungen betrachtet werden. So kann ein hydrophober Effekt bei der Komplexbildung dominieren. Es können aber auch andere Interaktionsformen zur Ausbildung einer Bindung beitragen, wie beispielsweise ionische Wechselwirkungen, Ion-Dipol-Wechselwirkungen, Dipol-Dipol-Wechselwirkungen, Wasserstoffbrückenbindungen, van der Waals-Kräfte, oder Dispersionskräfte. Schließlich kann neben den genannten Beispielen für nichtkovalente Verbindungen auch eine kovalente Verbindung von zu transportierender Substanz und prokaryontischem Transportprotein bewirkt werden. Dabei wird entweder eine Fusion auf Genebene durchgeführt, oder es wird eine chemisch stabile Atombindung zwischen zwei Atomen der Interaktionspartner ausgebildet.

Zusammenfassend weist das erfindungsgemäße Transferverfahren folgende Vorteile gegenüber dem Stand der Technik auf:
- signifikant höhere Effizienz als die bestehenden Verfahren;
- keine oder nur minimale Toxizität;
- keine oder nur geringe Immunogenität bei *in* vivo-Anwendungen;
- kostengünstig in der Herstellung und in der Lagerung.
- einfache und schnelle Anwendbarkeit;
- Transfer von Nukleinsäuren beliebigen chemischen Typs;
- Transfer von anderen, kovalent oder nichtkovalent gekoppelten Aminosäuren enthaltenden Substanzen wie Proteine oder Peptide;
- weitgehend keine Einschränkung bezüglich der Zielzellen (beispielsweise ist das Verfahren in gleicher Weise geeignet für eukaryontische tierische Zellen, eukaryontische pflanzliche Zellen und prokaryontische Zellen);
- Möglichkeit der variablen Zusammensetzung und der zusätzlichen Anheftung (Fusionierung) von Effektoren oder der Umhüllung und weitere Möglichkeiten zur Integration weiterer günstiger Eigenschaften für die Zellaufnahme.

Folgende Beispiele zeigen Anwendungen dieser Erfindung, sollen den Schutzumfang der Erfindung jedoch nicht einschränken.

In der Beschreibung und in den Beispielen wird auf die folgenden Figuren Bezug genommen.
**Figur 1** zeigt eine schematische Übersicht über mögliche Verwendungsverfahren der Erfindung beim Transport von Nukleinsäuren. Das TmHU-Protein (Y) vermittelt eine Kondensation der linearen oder zirkulären Nukleinsäure. Der Protein/Nukleinsäure-Komplex wird dann von Zellen aufgenommen (linke Figur). Als weitere Ausgestaltung kann beispielsweise eine rezeptorvermittelte Aufnahme (mittlere Figur) oder eine Umhüllung des Protein/Nukleinsäure-Komplexes erfolgen (rechte Figur).

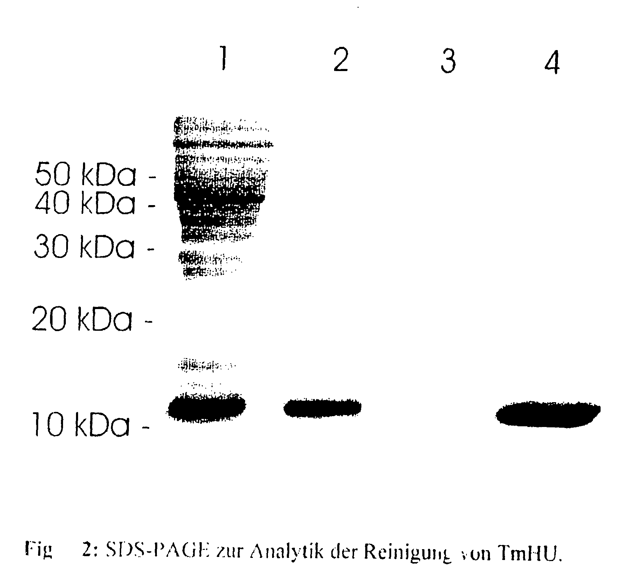

**Figur 2** zeigt eine SDS-PAGE zur Analytik der Reinigung von TmHU, das rekombinant in *Escherichia coli* hergestellt wird. Bahn 1 zeigt den löslichen Anteil des Zellextrakts von *E. coli.* Bahn 2 zeigt den Überstand nach Hitzefällung. Bahn 3 zeigt den Molekularmassenstandard (Größe ist links in der Figur angegeben). Bahn 4 zeigt das Eluat nach Reinigung über eine Kationenaustauschersäule. Das Protein kann mit sehr hoher Reinheit durch einfache und wenige Reinigungsschritte gewonnen werden.

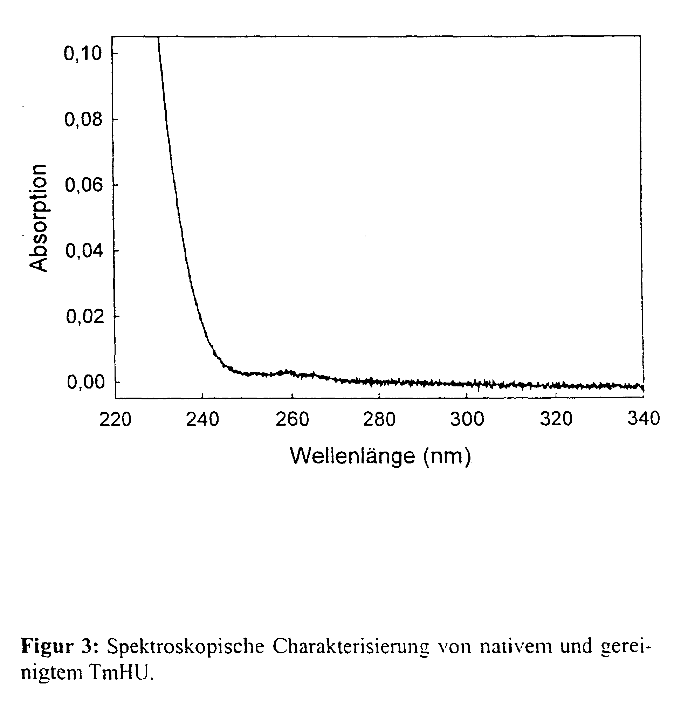

**Figur 3** zeigt die spektroskopische Charakterisierung von nativem und gereinigtem TmHU. Das UV-Absorptionsspektrum des Proteins (Konzentration 0.5 mg/ml) zeigt keine Absorption bei 280 oder 260 nm, die auf Kontaminationen durch Fremdproteine oder Nukleinsäuren schließen lassen würden; die Präparation ist damit sehr rein. Das Protein weist keine Tyrosin- oder Tryptophanreste auf und ist daher spektroskopisch transparent im angegebenen UV-Bereich. Die drei Phenylalaninreste der Proteinuntereinheiten zeigen - wie zu erwarten - eine geringfügige Absorption bei 257 nm.

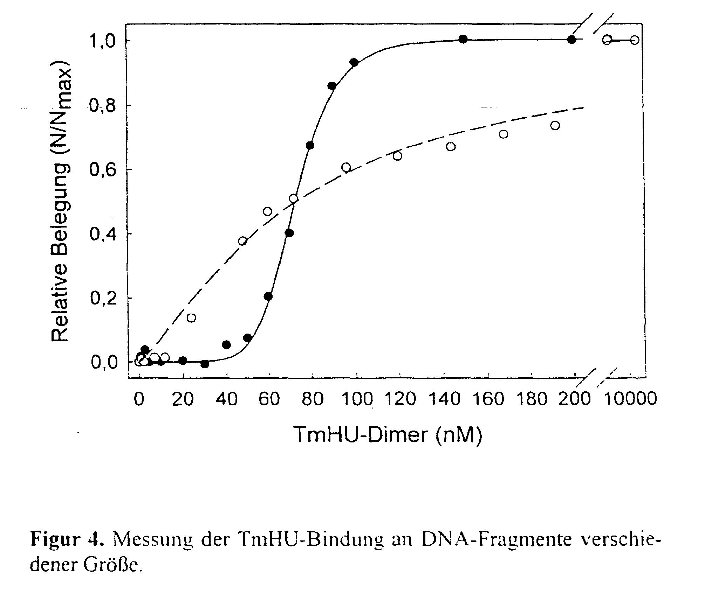

**Figur 4** zeigt die Messung der TmHU- Bindung an DNA-Fragmente verschiedener Größe mit Hilfe von Oberflächen-Plasmon-Resonanz. (•), 56 bp dsDNA-Fragment. und (―), berechnete Hill-Gleichung mit einem K_{D} von 73 nM und einem Hill-Koeffizienten von 7.6. (o), 23 bp dsDNA-Fragment, und (---), berechnete Hill-Gleichung mit einem K_{D} von 73 nM und einem Hill-Koeffizienten von 1.3. Die Bindung des Proteins an die DNA ist vergleichsweise stark und bei DNA-Fragmenten üblicher Größe (>> 23 bp) hoch kooperativ.

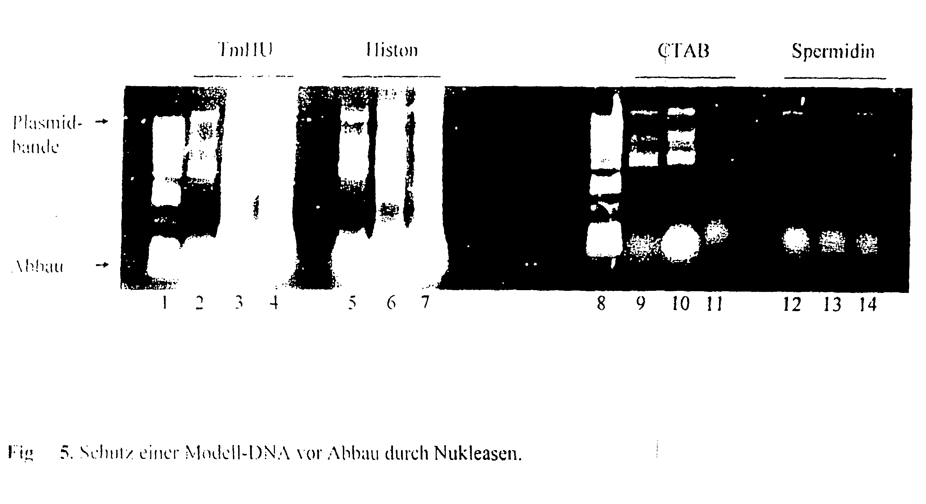

**Figur 5** zeigt den Schutz einer Modell-DNA vor Abbau durch Nukleasen. Es wurden verschiedene Konzentrationen unterschiedlicher Kondensations-Agentien verwendet. Bahn 1, Größenstandard, Bahn 2- 4, TmHU (0.3, 3, und 15 µg); Bahn 5 - 7, Histone (0.3, 3, und 30 µg); Bahn 8, Größenstandard; Bahn 9 - 11, CTAB (5, 10, und 100 µM); Bahn 12 - 14, Spermidin (10, 100, und 2000 µM). Unter den sehr stringenten Bedingungen wird ungeschützte DNA vollständig abgebaut, CTAB und Spermidin bieten dabei kaum Schutz vor diesem Abbau. Histone bieten etwas verbesserten Schutz, und TmHU bietet in diesem Vergleichsansatz den besten Schutz vor Abbau.

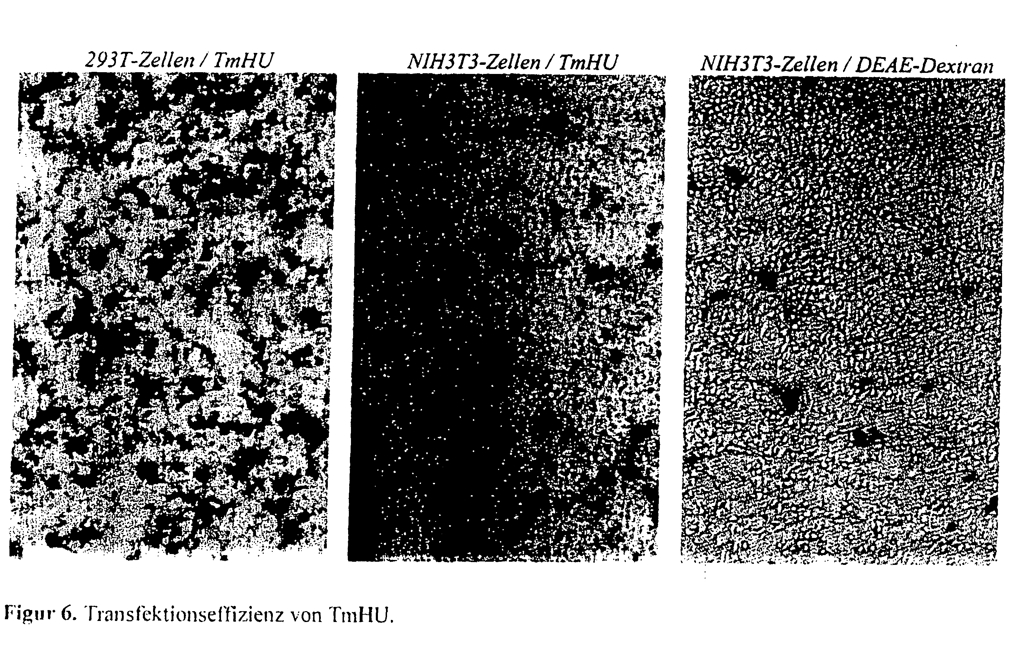

**Figur 6** zeigt die Transfereffizienz von TmHU mit verschiedenen Zellinien im Vergleich zu Standardmethoden (DEAE-Dextran-Transfektion). Links, Transfektion von humanen 293T-Zellen mit TmHU; die Transfektionseffizienz beträgt hier ca. 50 %. Mitte, Transfektion von murinen NIH3T3-Zellen mit TmHU; die Transfektionseffizienz beträgt hier etwa 30 %. Rechts, Transfektion von murinen NIH3T3-Zellen mit DEAE-Dextran; die Transfektionseffizienz beträgt hier bis zu 10%.

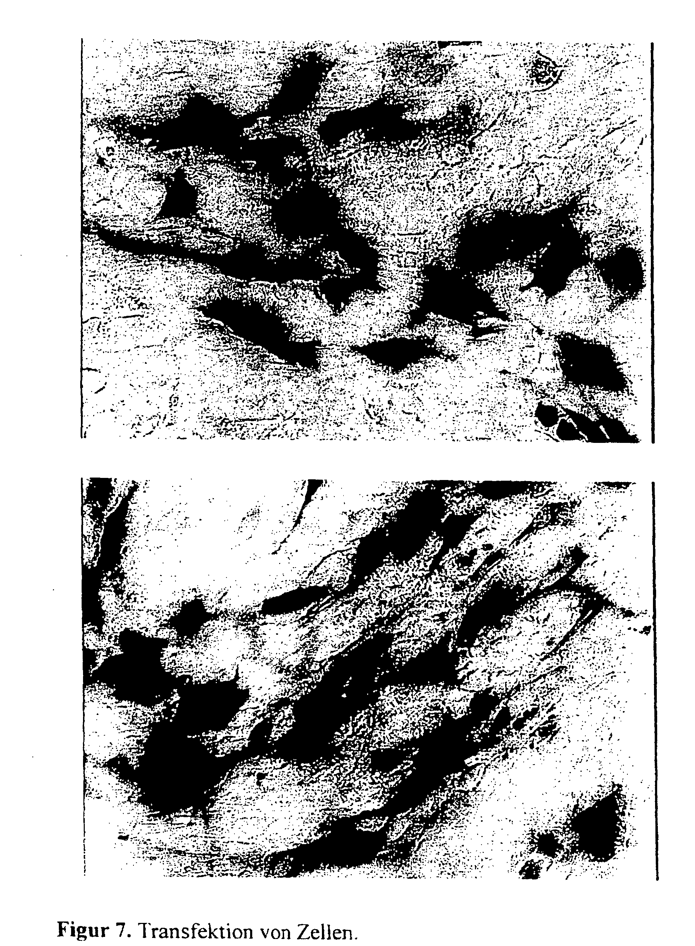

**Figur 7** zeigt die Transfereffizienz von TmHU mit NIH 3T3-Zellen mit dem in Beispiel 5 beschriebenen Protokoll. Es sind dabei zwei verschiedene Ausschnitte einer Zellkulturschalenvertiefung dargestellt; die mikroskopische Vergrößerung am Objektiv beträgt 20fach. Die Ausbeute an Transfektionsereignissen beträgt hier etwa 2500 positive Zellen pro µg an eingesetzter DNA.

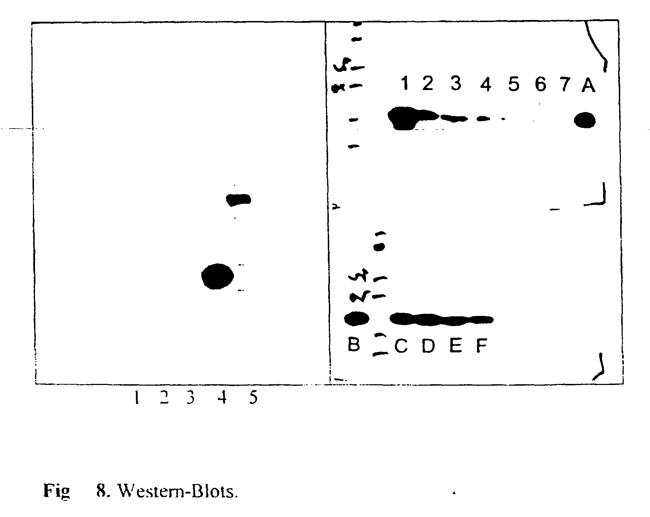

**Figur 8** zeigt Western-Blots zum Nachweis der Aufnahme des TmHU-EGF-Fusionsproteins in humane A431-Zellen, die als Tumormarker den EGF-Rezeptor auf der Zelloberfläche exprimieren. Linke Figur: Western-Blot der Proteine aus dem Zellysat nach Inkubation und Aufnahme des TmHU-EGF-Fusionsproteins. Bahn 1, Transfektion mit TmHU-EGF; Bahn 2, Transfektion mit TmHU-EGF/DNA-Komplex; Bahn 3, Lysat von untransformierten A431-Zellen zur Kontrolle der Spezifität des Anti-EGF-Antikörpers; Bahn 4, TmHU-EGF-Fusionsprotein (Standard); Bahn 5, Markierung des Molekulamzassenstandards. Rechte Abbildung: Zeitlicher Verlauf des Verschwindens von TmHU-EGF aus dem Zellüberstand im Verlauf der Transfektion; Nachweis durch Westernblot. Bahnen 1 bis 7: Inkubation mit DNA bei der Transfektion; Bahnen A bis G: Inkubation ohne DNA bei der Transfektion. Bahn 1, TmHU-EGF-Vergleichsstandard; Bahn 2, 0 min; Bahn 3, 30 min; Bahn 4, 60 min; Bahn 5, 90 min; Bahn 6, 120 min; Bahn 7, 7 h; Bahn A, 0 min; Bahn B, 30 min; Bahn C, 60 min; Bahn D, 90 min; Bahn E, 120 min; Bahn F: 7 h. Es ist deutlich die Aufnahme des TmHU in die Zellen zu beobachten, wobei durch die Ausbildung der TmHU-DNA-Komplexe eine effizientere Aufnahme bewirkt wird, als ohne diese Komplexbildung.

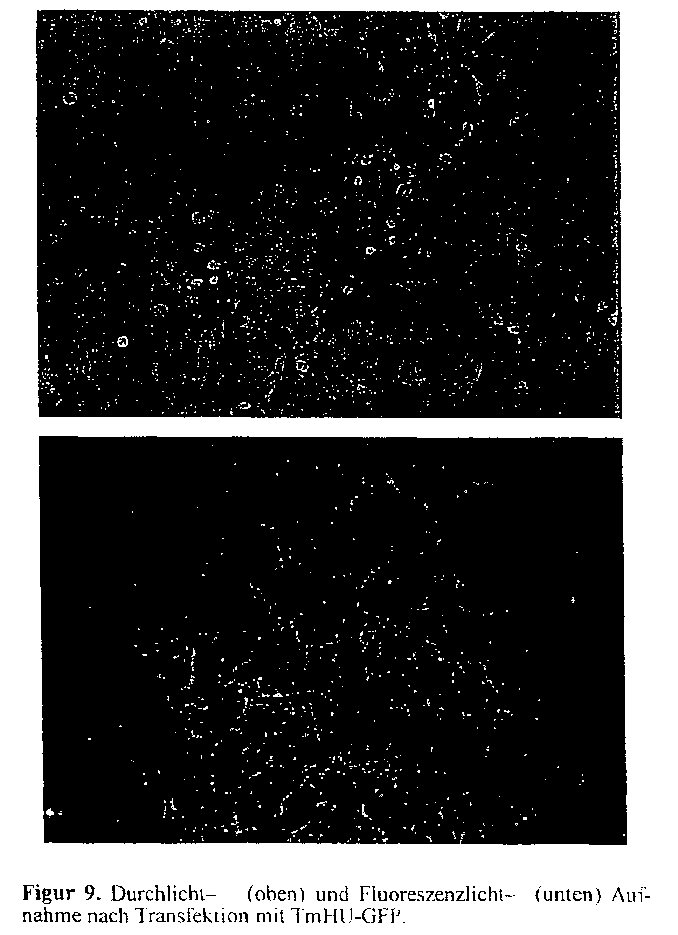

**Figur 9** zeigt eine Durchlichtaufnahme (oben) und eine Aufnahme unter Fluoreszenzlicht-Bedingungen (unten) eines Ausschnitts aus einer transfizierten Kulturschale von NIH 3T3-Zellen. Die Zellen wurden für 5 Stunden im Medium inkubiert und anschließend mit PBS gewaschen. Die grün leuchtenden Zellen enthalten das TmHU-GFP-Fusionsprotein, das vermittels des TmHU-Aufnahmemechanismus in die Zellen aufgenommen wurde.

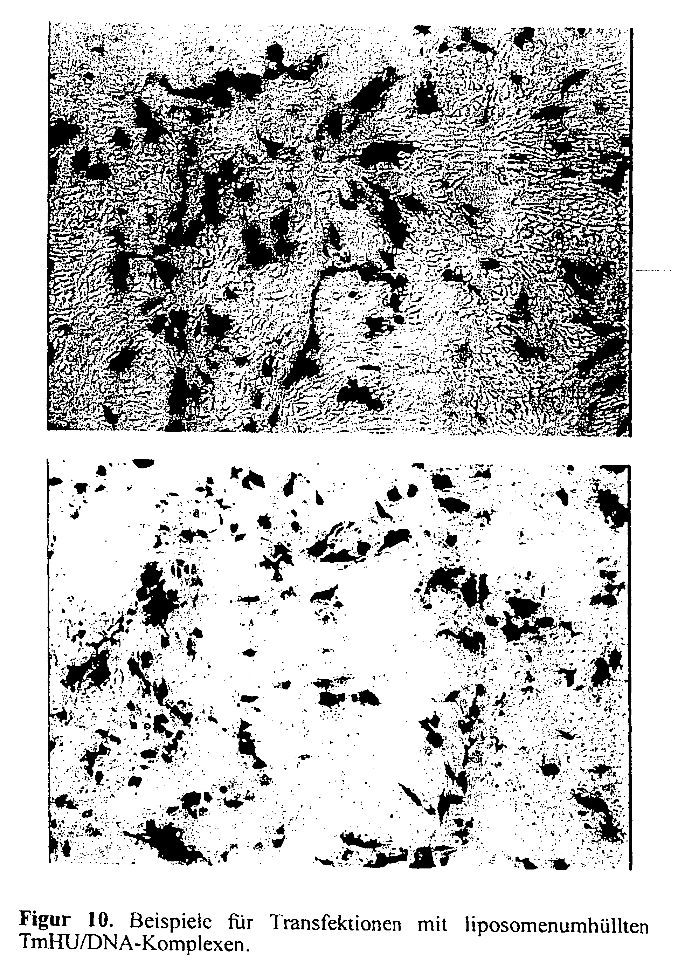

**Figur 10** zeigt beispielhaft zwei Zellkulturen mit NIH 3T3-Zellen, die mit liposomenumhüllten TmHU/DNA-Komplexen transfiziert wurden. Es sind in beiden Ansätzen eine große Zahl positiv transformierter Zellen zu verzeichnen, die das Reportergen exprimieren und dadurch eine Blaufärbung im Testansatz zeigen.

### Beispiel 1

### Klonierung, rekombinante Expression in Escherichia coli und Reinigung des Proteins HU von Thermotoga maritima

Nach dem Stand der Technik ist die heterologe Expression einer klonierten DNA-Sequenz in einer prokaryontischen Wirstzelle bekannt. Durch eine Polymerase- Kettenreaktion (PCR) mit den Oligonukleotid-Primern TmHU-N (5'-GGG GGT CAT ATG AAC AAA AAA GAA CTG ATC GAC AGG GTG G-3') und TmHU-C (5'-TTC CGG ATC CCT ATC ACT TGA CCT TCT CTT TGA GGG C-3') auf genomische DNA aus dem Organismus *Thermotoga maritima* kann ein 300 bp-Fragment amplifiziert und mit Standardtechniken (siehe dazu Sambrook *et al.,* "Molecular Cloning. A Laboratory Manual", 1989. Cold Spring Harbor Laboratory Press) in den prokaryontischen Expressionsvektor pET11a (Fa. Novagen) einkloniert werden. Nach Transformation des Plasmids in *E. coli*-Zellen des Stammes BL21(DE3) (Fa. Stratagene) und Selektion auf ampicillinresistenten LB-Agarplatten wird eine Vorkultur von LB-Medium, das 100 µg/ml Ampicillin enthält, mit einer einzelnen Kolonie angeimpft und über Nacht bei 37 °C geschüttelt. Diese Vorkultur wird im Verhältnis 1:100 in die Hauptkultur, bestehend aus dem gleichen Medientyp, verdünnt. Die Hauptkultur wird bei 37 °C im Schüttelschrank inkubiert, bis die Absorption der Bakteriensuspension bei einer Wellenlänge von 600 nm den Wert 1.0 erreicht. Die Expression des *Thermotoga maritima* HU-Gens (TmHU) wird anschließend durch Zugabe von 1 mM Isopropylthiogalactosid (IPTG) induziert.
Nach einer weiteren Wachstumszeit von 90 min werden die Zellen durch Zentrifugation (6000 g, 15 min, 4 °C) abgetrennt, in einem Resuspensionspuffer (50 mM Natriumphosphat, pH 7.5, 300 mM Natriumchlorid, 5 mM EDTA, 1 mM PMSF) aufgenommen und anschließend durch Hochdruckhomogenisierung aufgeschlossen. TmHU bleibt dabei vollständig im löslichen Überstand des Zellaufschlusses. Nach Zugabe von Benzonase (Fa. Merck) wird der Zellaufschluß für eine Stunde bei Raumtemperatur zur enzymatischen Beseitigung der störenden Nukleinsäuren (DNA und RNA) aus dem Wirtsorganismus inkubiert.

Nach Zentrifugation zur Trennung der löslichen von den unlöslichen Bestandteilen (50 000 g, 1 h, 4 °C) wird der Überstand für 20 min auf 80 °C erhitzt. Dabei wird der größte Teil der Wirtsproteine thermisch denaturiert und fällt nach Abkühlung als unlösliches Präzipitat aus. Das thermostabile Protein TmHU liegt nach erneuter Zentrifugation bei 50 000 g in angereicherter und bereits nahezu reiner Form im Überstand vor (Fig. 2).

Im nachfolgenden letzten Reinigungsschritt wird der Überstand durch eine Kationenaustauschchromatographie über eine Säule vom Typ Poros HS (Fa. Perseptive Biosystems) aufgereinigt. Unter den Bedingungen des Resuspensionspuffers bindet TmHU stark an die Kationentauschersäule. Nach Anlegen eines linearen Salzgradienten von 300 bis 2000 mM NaCl kann TmHU von den restlichen, noch verbliebenen Verunreinigungen chromatographisch separiert eluiert werden. Fig. 2 zeigt die Effizienz der einzelnen Reinigungsschritte mit Hilfe eines 18%igen SDS-Polyacrylamidgels. Durch das hier dargestellte Verfahren kann das unter Nativbedingungen als Dimer vorliegende TmHU in spektroskopisch reiner Form (> 95 % Reinheit; Fig. 3) mit einer Ausbeute von etwa 20 mg je Liter *E. coli-*Kultur (bei einfachem Anzuchtverfahren) gewonnen werden.

### Beispiel 2

### Nachweis der Nukleinsäure-bindenden Eigenchaften von TmHU

Ein 56 bp-DNA-Fragment sowie ein 23 bp-Fragment werden über jeweils 2 Primer, von denen je einer biotinyliert ist, mit PCR-Technik amplifiziert und die entstehende doppelsträngige DNA an Streptavidin-Chips immobilisiert. Mittels Oberflächen-Plasmonresonanz (SPR), bestimmt durch ein BIACore-Gerät (Fa. Amersham Pharmacia Biotech), kann die Bindung von TmHU an die jeweils immobilisierte DNA direkt gemessen werden (Fig. 4). Dabei wird TmHU in verschiedenen Konzentrationen in einem Puffer, der 50 mM Natriumphosphat. pH 7.5 und 100 mM Natriumchlorid enthält, in die Flußzelle des DNA-Chips injiziert und das SPR-Signal aufgezeichnet. Die Plateaus der Signale sind direkt proportional zur gebundenen Menge an TmHU. Eine Auftragung des SPR-Signals gegen die Konzentration des eingesetzten TmHU ergibt eine sigmoide Kurve gemäß der Hill-Gleichung (Fig. 4), die im Falle des 56 bp-Fragmentes charakteristisch für eine hoch kooperative Bindung ist. Der Übergangsmittelpunkt der Kurven, das ist definitionsgemäß die Dissoziationskonstante K_{D}, liegt dabei in beiden Fällen bei einer TmHU-Proteinkonzentration von 73 nM.

### Beispiel 3

### Schutz von Nukleinsäuren vor Abbau durch Nukleasen

1 µg einer zirkulären Plasmid-DNA (pEGFP-N1, Fa. Clontech) in 120 µl Probenpuffer (20 mM HEPES, pH 7.2, 100 mM NaCl, 5% Glycerol, 10 mM MgCl₂) wird mit TmHU, humanen Histonen (Fa. Sigma), Cetyl-Trimethyl-Ammoniumbromid (CTAB, Fa. Amresco) oder Spermidin (Fa. Sigma) jeweils in drei verschiedenen Konzentrationen versetzt, für eine Stunde bei Raumtemperatur inkubiert, und anschließend mit 5 Einheiten (units) Benzonase (Fa. Merck) versetzt. Nach 30 min Inkubation bei Raumtemperatur werden die Proben auf Konzentrationen von 0.5 % SDS. 20 mM EDTA, gebracht, die Nukleinsäuren werden mit Phenol-Chloroform extrahiert und aus der wäßrigen Phase mittels Ethanol gefällt. Anschließend wird nach Anfärben der DNA mit dem sensitiven Farbstoff SYBR Gold (Fa. Molecular Probes) auf einem 1 %igen Agarosegel der Schutz der Nukleinsäuren vor Nuklease-Abbau analysiert (Fig. 5). In diesem Beispiel wird der erhebliche Schutz der DNA durch TmHU vor dem Abbau durch Nukleasen deutlich. Die niedermolekularen Substanzen (CTAB und Spermidin) bieten nur einen schlechten Schutz vor Nukleaseabbau; dieser Schutz ist bei eukaryontischen Histonen deutlich verbessert (Fig. 5). Jedoch erst bei Verwendung des prokaryontischen TmHU-Proteins wird unter ansonsten identischen Versuchsbedingungen ein deutlicher Schutz der Nukleinsäuren vor Nukleaseabau erreicht. Ungeschützte DNA wird unter den Versuchsbedingungen vollständig abgebaut.

Der Schutz vor nukleaseinduziertem Abbau ist für die Optimierung von Transfereffizienzen wichtig, da im serumhaltigen Medium und teilweise auch in den Zielzellen die Nukleinsäuren einem Abbau durch Nukleasen ausgesetzt ist. Aus diesem Beispiel wird weiterhin deutlich. daß das prokaryontische Protein den eukaryontischen Histonen in dieser Hinsicht signifikant überlegen ist, vermutlich durch das Fehlen der für Eukaryonten charakteristischen repetitiven Strukturen in den Nukleohistonkomplexen, die eine Spaltung an den Verbindungsstellen begünstigen können. Dieser Schutz vor Abbau ist ein sehr günstiges Ausgestaltungsmerkmal für das in der vorliegenden Erfindung beschriebene Verfahren. Dazu kann dieser Schutz auch bei der Herstellung von Plasmiden *in vivo* dazu eingesetzt werden, um eine verbesserte Herstellung beispielsweise in Bakterien zu erreichen.

### Beispiel 4

### Transfektion von eukaryontischen Zellen

300 µl einer TmHU-Lösung (0.5 mg/ml) und 6 µl Plasmid-DNA (24 µg pCMV-β, kodiert für das Markerprotein β-Galaktosidase) werden zusammen für eine Stunde bei Raumtemperatur inkubiert. Anschließend wird diese Mischung in das Medium von halbkonfluenten murinen NIH3T3- oder humanen 293T-Zellen (60 mm-Zellkulturschale) gegeben. Die Schalen werden leicht geschwenkt und bei 37 °C und 5 % CO₂ für 2 Tage inkubiert; in dieser Zeit wird kein weiterer Medienwechsel durchgeführt. Dabei bildet sich ein Präzipitat aus Protein und Nukleinsäuren, das auf die adhärenten Zellen sedimentiert. Der Protein/Nukleinsäure-Komplex wird in dieser Zeit von den Zellen aufgenommen. Nach 48 h wird eine Anfärbung der transformierten Zellen mit einem Substrat für die β-Galaktosidase, 5-Bromo-4-chloro-3-indolyl-β-D-galactopyranosid (X-Gal), durchgeführt und das Verhältnis der gefärbten (transfizierten) zu den nicht gefärbten (nicht transfizierten) Zellen bestimmt. Daraus errechnet sich die Transfektionseffizienz.

Dazu werden zusätzlich Kontrolltransfektionen durchgeführt, in denen entweder jeweils die Nukleinsäuren oder das TmHU im Transfektionsansatz fehlen *(Negativkontrolle).* Als Vergleich zur Bestimmung der Effektivität wird parallel eine optimierte Transfektion mit DEAE-Dextran (Mammalian Transfection Kit. Fa. Stratagene) nach den Angaben des Herstellers durchgeführt *(Positivkontrolle).* Die Negativkontrollen zeigen - wie erwartet - jeweils keine gefärbten Zellen, also auch keine Transfektionen. Bei der Positivkontrolle (DEAE/Dextran-Transfektion) werden maximal 10 % der Zellen in der Kulturschale gefärbt (vgl. Fig. 6); dies liegt in der Größenordnung der aus der Literatur bekannten Effizienzen für dieses Verfahren. Eine Transfektion der 293T-Zellen mittels DEAE-Dextran-Verfahren ist nicht erfolgreich, da die nur schwach angehefteten Zellen durch das Transfektionsverfahren abgelöst werden. Im Gegensatz dazu ist eine Transfektion mit TmHU bei beiden Zellinien problemlos möglich, eine Ablösung der 293T-Zellen wird nicht beobachtet. Bei den TmHU-basierten Transfektionen betragen die Transfektionseffizienzen jeweils 30 % (murine NIH3T3-Zellen) bzw. 50 % (humane 293T-Zellen). Es ist also eine deutliche Steigerung der Transfektionseffizienzen der Zellen gegenüber einem Standardsystem zu beobachten (vgl. Fig. 6). Diese Ausbeuten erscheinen darüber hinaus durch Optimierungen noch weiter steigerbar. Bis zur Fixierung der Zellen (Abtötung) zur Anfärbung mit X-Gal war ein negativer Einfluß der TmHU-Transfektion auf das Überleben und das Wachstum der Zellen nicht zu beobachten; das TmHU ist mithin nicht toxisch für die Zellen.

### Beispiel 5

### Alternatives Protokoll zur Transfektion von eukaryontischen Zellen

100 µl einer TmHU-Lösung (0.5 mg/ml) in Phosphatpuffer, pH 7.0, und 1 µl einer Plasmid-DNA-Lösung (4 µg pCMV-β, kodierend für das Markerprotein β-Galaktosidase), werden mit 10 µl einer 20 mM CaCl₂- Lösung vermischt und für 40 min auf 95 °C erhitzt. Nach Abkühlen auf Raumtemperatur und Inkubation für weitere 20 min wird die Mischung mit 350 µl DMEM-Medium mit 10 % FBS versetzt und in die Vertiefung einer 12-well-Platte gegeben, in die 16 Stunden vorher 50 000 NIH 3T3-Zellen ausgesät wurden. Vor der Zugabe wird das Medium über den Zellen entfernt. Nach leichtem Schwenken wird die Schale für 12 h im Brutschrank bei 37 °C und 5 % CO₂ inkubiert. Das Medium wird anschließend gegen jeweils 1 ml frisches Vollmedium (DMEM mit 10 % FBS) ausgetauscht und die Zellen für weitere 36 h inkubiert. Es wird nun der im Beispiel 4 beschriebene Test auf β-Galaktosidase-Expression durchgeführt und die Zellen mit Hilfe eines gerasterten Okulars gezählt. Typischerweise erhält man mit dieser Methode etwa 10 000 positive Zellen pro Well (Beispiele in Fig. 7). Wird das beschriebene Protokoll ohne die Zugabe von TmHU zum Transfektionsansatz durchgeführt (Kontrolle auf Effizienz einer Standard-Calciumphosphat-Transfektion zum Vergleich), so erhält man ca. 10 Transformanden, also eine um einen Faktor 10³ geringere Ausbeute. Die Ausbeute an Transfektionsereignissen ist im vorliegenden Beispiel mit ca. 2 500 positiven Zellen pro µg DNA etwa 5- bis 10mal höher als die einer DEAE-Dextran-Transfektion (etwa 300 bis 400 Transfektionsereignisse pro µg DNA).

### Beispiel 6

### Verwendung modifizierter Proteine am Beispiel von TmHU-EGF

TmHU-EGF ist ein Fusionsprotein aus TmHU und dem humanen epidermalen Wachstumsfaktor (EGF), der eine Aufnahme in EGF-Rezeptor tragende Zielzellen, wie beispielsweise die hier verwendeten humanen A431-Zell (vgl. E.J. Meuillet *et al.,* "Sialidase gene transfection enhances epidermal growth factor receptor activity in an epidermoid carcinoma cell line, A431"; Cancer Res. 1999. Band 59, S. 234-240), ermöglichen sollte. Das Fusionsprotein wird im Anschluß an eine Klonierung über SacII-Schnittstellen in den in Beispiel 1 genannten pET11a-Expressionsvektor nach dem in Beispiel 1 beschriebenen Standardverfahren hergestellt. 100 µl TmHU-EGF (0.5 mg/ml) werden mit 8 µg des pCMV-β-Plasmids für 1 Stunde inkubiert und der entstehende Komplex aus TmHU-EGF/DNA gemäß den Angaben von Beispiel 4 in das Medium über halbkonfluente A431-Zellen gegeben. Als eine Kontrolle werden 100 µl TmHU-EGF ohne zusätzliche DNA auf identisch behandelte Zellen gegeben. In einer Negativkontrolle wird außerdem eine halbkonfluente Zellkulturschale vorbereitet, in die kein TmHU-EGF gegeben wird.

Die Zellen werden jeweils für 2 Stunden auf Eis inkubiert, dann mit kaltem PBS-Puffer gewaschen und anschließend bei 37 °C für 45 min inkubiert. Das Medium über den Zellen wird entfernt und Trypsin/EDTA-Lösung zum Ablösen des bis dahin nicht internalisierten Komplexes sowie der adhärenten Zellen zugegeben. Der Trypsinverdau wird durch Zugabe von FCS-haltigem Medium (FCS: Fötales Kälberserum) gestoppt und die resuspendierten Zellen dreimal mit kaltem PBS-Puffer gewaschen. Nach dem letzten Waschschritt werden die Zellen in 100 µl PBS-Puffer resuspendiert und anschließend mit 100 µl 4 % SDS und 2 mM PMSF versetzt, kräftig gemischt und sofort für 10 min auf 95 °C erhitzt. Die so hergestellten Zellysate werden auf ein 15 %iges SDS-Polyacryamidgel aufgetragen und nach der Elektrophorese mittels Western-Blot analysiert (Erstantikörper: rabbit polyclonal anti-EGF, Fa. Santa Cruz: Zweitantikörper: goat anti-rabbit IgG, HRP-conjugate, Fa. BioRad).

Im spezifischen Western-Blot (Fig. 8. links) kann man in den Lysaten, die mit der TmHU-EGF-Chimäre inkubiert werden, das Protein als spezifische Bande nachweisen; bei Lysaten ohne TmHU-EGF-Inkubation fehlt diese Bande. Damit ist gezeigt, daß das Protein TmHU-EGF in die A431-Zellen internalisiert wird und diese Aufnahme mittels spezifischem Western Blot nachweisbar ist. Diese Aufnahme ist unabhängig von der Anwesenheit von DNA im Experiment, jedoch ist die Aufnahme in Gegenwart von DNA effizienter, dies wird durch ein zweites Experiment unterstützt, das analog zum vorstehend beschriebenen Transfektionsexperiment durchgeführt wird (vgl. Fig. 8, rechts). Hier wird die Aufnahme der TmHU-EGF-Chimären in die A431-Zielzellen durch die Abnahme der TmHU-Menge im Medium über den Zellen detektiert. Auch hier zeigt sich, daß DNA-beladenes TmHU-EGF erheblich schneller aus dem Zellüberstand abgezogen wird, als im Kontrollexperiment ohne DNA.

### Beispiel 7

### Verwendung modifizierter Proteine am Beispiel von TmHU-GFP

TmHU-GFP ist ein Fusionsprotein aus TmHU und dem Green Fluorescent Protein der Tiefseequalle *Aequorea victoria,* das die bemerkenswerte Eigenschaft aufweist, ohne Kofaktoren, also nur aufgrund seiner Tertiärstruktur, grün zu fluoreszieren. Fusionskonstrukte mit GFP behalten oftmals diese Fluoreszenz des nativen Proteins bei. In dem vorliegenden Beispiel ist das GFP C-terminal an das TmHU über einen Linker fusioniert. Die Fusion wurde auf DNA-Ebene durchgeführt, durch gentechnische Standardmethoden wurde dabei das GFP-Gen mitsamt einem Linker aus dem Vektor pEGFP-N1 der Firma Clontech ausgeschnitten und am 3'-Ende des TmHU-Gens eingefügt. Die Reinigung von TmHU-GFP erfolgt analog zu der des TmHU aus Beispiel 1, über einen Kationentauscher. Das Fusionsprotein kann löslich in hoher Ausbeute gereinigt werden.

TmHU-GFP besitzt, wie GFP selbst auch, eine grünliche Fluoreszenz. Ebenso bleibt die DNA-Bindung des Fusionsproteins erhalten, denn bei Zugabe von hochmolekularer DNA zur Proteinlösung und anschließender Zentrifugation wird TmHU-GFP mit der DNA kopräzipitiert und zeigt grün leuchtende Komplexe. Es kann somit davon ausgegangen werden, daß beide Anteile des Fusionskonstrukts funktionell erhalten sind. Dies ist ein weiterer Hinweis dafür, daß das System leicht auf genetischer Ebene modifizierbar ist und die Struktur von TmHU ein stabiles Gerüst zur Erschaffung weiterer Varianten in Form von Fusionskonstrukten darstellt.

Mit der vorliegenden Variante TmHU-GFP lassen sich nun Aufnahmestudien in eukaryontische Zellen *in* vivo durchführen, also ohne eine Fixierung der Zellen. Dazu wird eine 2 cm-Kulturschale mit NIH 3T3-Zellen mit PBS-Puffer gespült und dann 50 µl TmHU-GFP-Lösung (0.1 mg/ml) in PBS-Puffer zugegeben. Nach kurzem Schwenken werden die Schalen für 5 Stunden bei 37 °C und mit 5 % CO₂ inkubiert und anschließend nach dreimaligem Spülen mit PBS mit einem Fluoreszenzmikroskop mit Digitalkamera Fotos der GFP-Fluoreszenz aufgenommen. Man erkennt in Fig. 9 deutlich die Umrisse der Zellen in der Fluoreszenz (unten) und im Vergleich mit einer Phasenkontrastaufnahme (oben) desselben Ausschnitts. Diese Befunde deuten darauf hin, daß das Fusionskonstrukt mit großer Effizienz in die Zellen aufgenommen wird und dabei nicht in einzelnen Vesikeln wie beispielsweise Lysosomen konzentriert und angereichert wird, sondern daß das Fusionsprotein im gesamten Cytosol vorliegt.

Aus der Literatur ist bekannt, daß diese Eigenschaft der Zellaufnahme nicht durch das GFP verursacht ist, sie muß also durch den TmHU-Teil des Fusionskonstruktes vermittelt worden sein.

Das Beispiel verdeutlicht damit, daß prokaryontische HU-Proteine wie TmHU mit hoher Effizienz in Zellen aufgenommen werden und dabei nicht in den Lysosomen angereichert werden, sondern sich im Cytosol verteilen. Diese Aufnahmeeigenschaft kann die hohe Gentransfereffizienz von TmHU erklären. Dabei tragen sowohl die effiziente Aufnahme in Zellen als auch das potentielle Freisetzen aus Endosomen und anderen zellulären Kompartimenten zur hohen Transfektionseffizienz bei. Das Beispiel verdeutlicht zudem, daß auch an TmHU angehängte oder fusionierte Proteine (beispielsweise auch therapeutisch relevante Proteine) mit hoher Effizienz durch TmHU in eukaryontische Zellen eingeschleust werden können. TmHU wirkt hier als Transportvehikel zum Durchqueren der Zellmembran für das Fusionsprotein.

### Beispiel 8

### Transfektion mit HU aus Escherichia coli (EcoHU)

100 µl einer EcoHU-Lösung (HU-Protein aus *Escherichia coli),* 0.5 mg/ml, werden mit 1 µl Plasmid-Lösung (4 mg/ml, kodierend für β-Galactosidase) vermischt und für 40 min auf 95 °C erhitzt, dann für 20 min bei Raumtemperatur inkubiert. Die Mischung wird in 350 µl DMEM und 10 % FBS mit 1 % PS aufgeschlämmt und anschließend auf NIH 3T3-Zellen gegeben (50 000 Zellen in einer Vertiefung einer 12-Well-Platte). Nach 2 Tagen wird die Zellkultur auf Expression von β-Galaktosidase untersucht. Unter diesen Bedingungen zeigen 104 Zellen β-Galaktosidase-Expression. Dies ist eine erheblich geringere Ausbeute als im Falle der analogen Verwendung von TmHU (etwa 600 positiv transformierte Zellen). Das Beispiel zeigt, daß eine Transfektion unter den in der vorliegenden Erfindung gemachten Bedingungen im Prinzip auch mit HU-Proteinen anderer Bakterien, in diesem Fall eines mesophilen Bakteriums, möglich ist.

### Beispiel 9

### Verpackung des TmHU-DNA-Komplexes in Liposomen und Transfektion mit diesen Liposomen-TmHU-DNA-Komplexem

Eine der neueren Methoden zum Gentransfer in eukaryontische Zellen besteht in der Verpackung der DNA in kationische Lipidvesikel, die dann mit der Zellmembran fusionieren und die DNA auf diese Art in die Zelle einbringen. Dabei kommt es allerdings zu geringeren Ausbeuten, als nach der effizienten Aufnahme in die Zellen zunächst zu erwarten wäre, denn oftmals werden diese Lipidvesikel in den Endosomen angereichert und es kommt nicht zu einer Überführung der DNA in das Cytoplasma und nachfolgend zum Transport in den Zellkern. In diesem Beispiel wird untersucht, inwiefern sich liposomenvermittelte und TmHU-vermittelte Transfektionen synergistisch kombinieren lassen.

Zunächst wurde eine Transfektion mit dem Reagenz Tfx-50 (Fa. Promega) nach Herstellerangaben durchgeführt. Dabei war ein Ladungsverhältnis von positiven Ladungen (Lipid) zu negativen Ladungen (DNA) von 2:1 am effizientesten (Optimierung nach Herstellerangaben). Es wurden mit der Methode 80 % der konfluent ausgesäten NIH 3T3-Zellen transfiziert mit 1 µg Plasmid-DNA (pCMV-β), 3 µl Tfx-50-Reagenz und 200 µl DMEM. Nach einstündiger Inkubation wird 1 ml Vollmedium (DMEM mit 10 % FBS) zugesetzt. Es wurden hier im Schnitt 3 300 positive (transformierte) Zellen pro µg DNA erhalten. Diese Transfektionseffizienz liegt in der gleichen Größenordnung wie die optimierte TmHU-Transfektion (vgl. Beispiel 5).

Im nächsten Schritt wird die Transfektion unter Zusatz von verschiedenen Mengen TmHU bei variierender Lipidmenge durchgeführt. Dabei werden die höchsten Transfektionsergebnisse durch eine einstündige Inkubation von 1 µg DNA mit 12.5 µl TmHU-Lösung (0.5 mg/ml) und einem Ladungsverhältnis von 4: 1 (bezüglich Lipid:DNA) erreicht (4.5 µl Lipidsuspension, 12.5 µl TmHU-Lösung, und 1 µg DNA auf 200 µl der Transfektionslösung). Die Anzahl der positiven Zellen pro µg eingesetzter DNA steigt dabei auf durchschnittlich 16 000 transformierte Zellen. Die Ausbeute einer kombinierten TmHU-Lipofektion ist damit etwa fünfbis sechsmal höher als die optimierten Protokolle der Lipofektion, sowie der TmHU-Transfektion alleine. Zwei Beispiele für auf diese Art transformierte Zellen sind in Fig. 10 gezeigt.

Obwohl nicht auszuschließen ist, daß in diesem Experiment auch TmHU-vermittelte Transfektion und Lipofektion parallel zueinander stattfinden, kann in jedem Falle ein deutlicher synergistischer Effekt verzeichnet werden, denn die Effizienz ist erheblich höher als die Summe der jeweiligen Einzelbeiträge (zumal sowohl vom Standpunkt der Lipofektion als auch vom Standpunkt der TmHU-Transfektion im angegebenen Experiment unter jeweils suboptimalen Bedingungen gearbeitet wird). Die wahrscheinlichste Erklärung für die hohe Effizienz ist daher die, daß die entstehenden TmHU-DNA-Komplexe zumindest teilweise in die entstehenden Liposomen des Lipofektionsagens eingeschlossen werden. Diese Umhüllung der TmHU-DNA-Komplexe mit einer Liposomenmembran zeigt auch eine deutlich erhöhte Effizienz gegenüber Standardmethoden wie DEAE-Dextran-Transfektion.

## Patentansprüche

1. Verfahren zum Transfer von Nukleinsäuren und/oder Nukleinsäureanaloga, und/oder Nukleinsäuren und/oder Nukleinsäureanaloga und/oder Aminosäuren enthaltenden Substanzen, in prokaryontische und/oder eukaryontische Zellen, wobei
- die zu transferierende Nukleinsäure oder das Nukleinsäureanalogon, oder die Nukleinsäuren oder Nukleinsäureanaloga oder Aminosäuren enthaltende Substanz mit einem prokaryontischen HU-Protein in Kontakt gebracht wird, um einen Komplex aus der Nukleinsäure, dem Nukleinsäureanalogon oder der Nukleinsäuren oder Nukleinsäureanaloga oder Aminosäuren enthaltenden Substanz und dem prokaryontischen Hu-Protein zu bilden,
und
- anschließend der Komplex aus Nukleinsäure, Nukleinsäureanalogon und/oder Nukleinsäuren oder Nukleinsäureanaloga oder Aminosäuren enthaltender Substanz und dem prokaryontischen HU-Protein mit den prokaryontischen oder eukaryontischen Zielzellen in Kontakt gebracht wird, um einen Transfer des Komplexes in die Zellen zu erreichen.

2. Verfahren nach Anspruch 1,
**dadurch gekennzeichnet,**
**daß** als Nukleinsäure einzelsträngige oder doppelsträngige DNA, einzelsträngige oder doppelsträngige RNA, DNA in Form von Plasmiden, Chromosomenfragmente, Antisense-RNA, Ribozyme, katalytische RNA, Nukleotide, chromosomale DNA oder kodierende mRNA transferiert wird.

3. Verfahren nach Anspruch 1 oder 2,
**dadurch gekennzeichnet**,
daβ als Nukleinsäure eine DNA mit kodierender Sequenz eingesetzt wird, die in den Zielzellen zur Expression gebracht wird.

4. Verfahren nach einem oder mehreren der vorhergehenden Ansprüche,
**dadurch gekennzeichnet,**
**daß** die eingesetzte Nukleinsäure durch das Inkontaktbringen mit dem prokaryontischen HU-Protein kompaktiert und vor Abbau geschützt wird.

5. Verfahren nach Anspruch 1,
**dadurch gekennzeichnet,**
**daß** als Nukleinsäureanalogon PNA (Peptide Nucleic Acid) transferiert wird.

6. Verfahren nach einem oder mehreren der Ansprüche 1 bis 5,
**dadurch gekennzeichnet,**
**daß** der Komplex aus Nukleinsäure oder Nukleinsäureanalogon und prokaryontischem HU-Protein reversibel ist.

7. Verfahren nach einem oder mehreren der Ansprüche 1 bis 5 zum Transfer von DNA in Pflanzenzellen oder tierische Zellen oder menschliche Zellen.

8. Verfahren nach Anspruch 1,
**dadurch gekennzeichnet,**
**daß** als Aminosäuren enthaltende Substanzen Proteine, Peptide, Peptidhormone, Enzyme, Proteindomänen, Glykoproteine, pharmazeutische Wirkstoffe auf Aminosäurebasis, Peptid-Hormone oder Lipoproteine transferiert werden.

9. Verfahren nach Anspruch 8,
**dadurch gekennzeichnet,**
**daß** die Komplex-Bildung aus Aminosäuren enthaltender Substanz und Prokaryontischem HU-Protein durch kovalente oder nichtkovalente Bindung erfolgt.

10. Verfahren nach Anspruch 8 oder 9,
**dadurch gekennzeichnet,**
**daß** als Aminosäuren enthaltende Substanz das Green Fluorescent Protein (GFP) transferiert wird.

11. Verfahren nach einem oder mehreren der vorhergehenden Ansprüche,
**dadurch gekennzeichnet,**
**daß** die prokaryontischen HU-Proteine aus kryophilen, mesophilen, thermophilen oder hyperthermophilen Organismen stammen.

12. Verfahren nach einem oder mehreren der vorhergehenden Ansprüche,
**dadurch gekennzeichnet,**
**daß** als prokaryontisches HU-Protein TmHU (*Thermotoga maritima* HU-Protein), Sso7d (aus *Sulfolobus solfataricus* und *Sulfolobus acidocaldarius*), Ssh7 (aus *Sulfolobus shibatae),* Sac7d (aus *Sulfolobus solfataricus* und *Sulfolobus acidocaldarius*), BstHU (aus *Bacillus stearothermophilus*), HU (aus *Escherichia coli*), IHF (aus *Escherichia coli*), BsuHU (aus *Bacillus subtilis),* SaHU (aus *Sulfolobus acidocaldarius),* BbuHU (aus *Borrelia burgdorferi),* BgaHU (aus *Borrelia garinii),* Baf-HU (aus *Borrelia afzelii),* IHF (aus *Pseudomonas aeruginosa*), DNA-bindendes Protein (*Chlamydia spp.*), Hsa (aus *Staphylococcus aureus*), RIHU (aus *Rhizobium leguminosarum*), HSl (aus *Strep-tomyces lividans),* HCj (aus *Campylobacter jejuni),* HU (aus *Bacillus caldolyticus),* HU (aus *Bacillus caldotenax),* HU (aus *Bacillus globigii),* HCc (aus *Caulobacter crescentus),* DNA-bindendes Protein (aus *Deinococcus radiodurans),* HSa (aus *Sulfolobus acidocalda*rius), Histon-ähnliches, DNA-bindendes Protein (aus *Streptococcus gordonii),* Histon-ähnliches, DNA-bindendes Protein (aus *Streptococcus mutans),* Histon-ähnliches, DNA-bindendes Protein (aus *Streptococcus pyogenes),* Histon-ähnliches, DNA-bindendes Protein (aus *Streptococcus thermophilus),* Histon-ähnliches, DNA-bindendes Protein (aus *Haemophilus influenzae Rd),* Histon-ähnliches, DNA-bindendes Protein (aus *Listeria monocytogenes),* Histon-ähnliches, DNA-bindendes Protein (aus *Serratia marcescens),* Histon-ähnliches, DNA-bindendes Protein (aus *Salmonella typhimurium),* Histon-ähnliches, DNA-bindendes Protein (aus *Thermus aquaticus),* Histon-ähnliches, DNA-bindendes Protein (aus *Rhizobium meliloti),* Histon-ähnliches, DNA-bindendes Protein (aus *Pseudomonas putida),* Histon-ähnliches, DNA-bindendes Protein (aus *Mycobacterium tuberculosis),* Histon-ähnliches, DNA-bindendes Protein (aus *Mycobacterium leprae),* Histon-ähnliches, DNA-bindendes Protein (aus *Zymomonas mobilis),* Histon-ähnliches, DNA-bindendes Protein (aus *Yersinia pseudotuberculosis),* Histon-ähnliches, DNA-bindendes Protein (aus *Mycobacterium bovis),* Histon-ähnliches, DNA-bindendes Protein (aus *Mycoplasma hyopneumoniae),* Histon-ähnliches, DNA-bindendes Protein (aus *Mycobacterium smegmatis),* Histon-ähnliches, DNA-bindendes Protein (aus *Helicobacter pylori),* Histon-ähnliches, DNA-bindendes Protein (aus *Aquifex aeolicus),* Histon-ähnliches, DNA-bindendes Protein (aus *Agrobacterium tumefaciens),* Histon-ähnliches, DNA-bindendes Protein (aus *Pseudomonas aeruginosa),* Histon-ähnliches, DNA-bindendes Protein (aus *Helicobacter pylori),* Histon-ähnliches, DNA-bindendes Protein (aus *Xanthomonas campestris),* Histon-ähnliches, DNA-bindendes Protein (aus *Vibrio proteolyticus),* Histon-ähnliches, DNA-bindendes Protein (aus *Streptomyces lividans),* Histon-ähnliches, DNA-bindendes Protein (aus *Rickettsia prowazekii),* Histon-ähnliches, DNA-bindendes Protein (aus *Streptomyces coelicolor),* Histon-ähnliches, DNA-bindendes Protein (aus *Mycoplasma capricolum),* Histon-ähnliches, DNA-bindendes Protein (aus *Borrelia burgdorferi),* Histon-ähnliches, DNA-bindendes Protein (aus *Borrelia japonica),* Histon-ähnliches, DNA-bindendes Protein (aus *Borrelia andersonii),* oder HTa (aus *Thermoplasma acidophilum),* eingesetzt wird.

13. Verfahren nach einem oder mehreren der vorhergehenden Ansprüche,
**dadurch gekennzeichnet,**
**daß** das prokaryontische HU-Protein derart modifiziert ist, daß eine erhöhte oder veränderte Aufnahmeeffizienz in die Zielzellen erfolgt.

14. Verfahren nach einem oder mehreren der vorhergehenden Ansprüche,
**dadurch gekennzeichnet,**
**daß** das prokaryontische HU-Protein derart modifiziert ist, daß eine erhöhte Freisetzung der transferierten Nukleinsäure, des Nukleinsäureanalogons oder der Nukleinsäuren oder Nukleinsäureanaloga oder Aminosäuren enthaltenden Substanz aus den Endosomen der Zielzellen erreicht wird.

15. Verfahren nach einem oder mehreren der vorhergehenden Ansprüche,
**dadurch gekennzeichnet,**
**daß** das prokaryontische HU-Protein derart modifiziert ist, daß der Komplex aus Nukleinsäure, Nukleinsäureanalogon oder der Nukleinsäuren oder Nukleinsäureanaloga oder Aminosäuren enthaltenden Substanz und dem prokaryontischen HU-Protein an ein Rezeptormolekül auf der Oberfläche der Zielzellen bindet.

16. Verfahren nach einem oder mehreren der vorhergehenden Ansprüche,
**dadurch gekennzeichnet,**
**daß** vor der Inkubation des Komplexes aus Nukleinsäure, Nukleinsäureanalogon oder der Nukleinsäuren oder Nukleinsäureanaloga oder Aminosäuren enthaltenden Substanz und dem prokaryontischen HU-Protein mit den Zielxellen eine Umhüllung des Komplexes mit einer Liposomen-Membran oder mit Polyethylenglykol erfolgt.

## Claims

1. A method for the transfer of nucleic acids and/or nucleic acid analogs, and/or nucleic acids and/or nucleic acid analogs and/or substances containing amino acids, into prokaryotic and/or eukaryotic cells, where
- the nucleic acid or the nucleic acid analog to be transferred, or the nucleic acids or nucleic acid analogs or substances containing amino acids are brought into contact with a prokaryotic HU-protein, in order to form a complex from the nucleic acid, the nucleic acid analog or the nucleic acids or nucleic acid analogs or substances containing amino acids and the prokaryotic HU-protein,
and
- subsequently, the complex of nucleic acid, nucleic acid analog and/or nucleic acids or nucleic acid analogs or substances containing amino acids and the prokaryotic HU-protein are brought into contact with the prokaryotic or eukaryotic target cells, in order to achieve a transfer of the complex into the cells.

2. The method according to claim 1,
**characterized in that**
as nucleic acid single-stranded or double-stranded DNA, single-stranded or double-stranded RNA, DNA in the form of plasmids, chromosome fragments, antisense RNA, ribozymes, catalytic RNA, nucleotides, chromosomal DNA or coding mRNA is transferred.

3. The method according to claim 1 or 2,
**characterized in that**
as nucleic acid a DNA with a coding sequence is employed, which is brought to expression in the target cells.

4. The method according to one or more of the preceding claims,
**characterized in that**
the employed nucleic acid is compacted and protected against degradation by being brought into contact with the prokaryotic HU-protein.

5. The method according to claim 1,
**characterized in that**
as nucleic acid analog PNA (Peptide Nucleic Acid) is transferred.

6. The method according to one or more of claims 1 to 5,
**characterized in that**
the complex of nucleic acid or nucleic acid analog and prokaryotic HU-profein is reversible.

7. The method according to one or more of claims 1 to 5 for the transfer of DNA in plant cells or animal cells or human cells.

8. The method according to claim 1,
**characterized in that**
as amino acid-containing substances proteins, peptides, peptide hormones, enzymes, protein domains, glycoproteins, pharmaceutical active compounds based on amino acids, peptide hormones or lipoproteins are transferred.

9. The method according to claim 8,
**characterized in that**
the complex formation of amino acid-containing substance and prokaryotic HU-protein occurs by covalent or non-covalent binding.

10. The method according to claims 8 or 9,
**characterized in that**
as amino acid-containing substance the Green Fluorescent Protein (GFP) is transferred.

11. The method according to one or more of the preceding claims,
**characterized in that**
the prokaryotic HU-proteins originate from cryophilic, mesophilic, thermophilic, or hyperthermophilic organisms.

12. The method according to one or more of the preceding claims,
**characterized in that**
as procaryotic HU-protein TmHU *(Thermotoga maritima* HU-protein), Sso7d (from *Sulfolobus solfataricus and Sulfolobus acidocaldarius), Ssh7* (from *Sulfolobus shibatae),* Sac7d (from *Sulfolobus solfataricus* and *Sulfolobus acidocaldarius),* BstHU (from *Bacillus stearothermophilus),* HU (from *Escherichia coli),* IHF (from *Escherichia coli),* BsuHU (from *Bacillus subtilis),* SaHU (from *Sulfolobus acidocaldarius),* BbuHU (from *Borrelia burgdorferi),* BgaHU (from *Borrelia garinii),* BafHU (from *Borrelia afzelii),* IHF (from *Pseudomonas aeruginosa*)*,* DNA-binding protein (*Chlamydia spp.*)*,* Hsa (from *Staphylococcus aureus*), RIHU (from *Rhizobium leguminosarum),* HSI (from *Streptomyces lividans),* HCj (from *Campylobacter jejuni),* HU (from *Bacillus caldolyticus),* HU (from *Bacillus caldotenax),* HU (from *Bacillus globigii),* HCc (from *Caulobacter crescentus),* DNA-binding protein (from *Deinococcus radiodurans),* HSa (from *Sulfolobus acidocaldarius),* histone-like, DNA-binding protein (from *Streptococcus gordonii),* histone-like, DNA-binding protein (from *Streptococcus mutans),* histone-like, DNA-binding protein (from *Streptococcus pyogenes),* histone-like, DNA-binding protein (from *Streptococcus thermophilus),* histone-like, DNA-binding protein (from *Haemophilus influenzae Rd),* histone-like, DNA-binding protein (from *Listeria monocytogenes),* histone-like, DNA-binding protein (from *Serratia marcescens),* histone-like, DNA-binding protein (from *Salmonella typhimurium),* histone-like, DNA-binding protein (from *Thermus aquaticus),* histone-like, DNA-binding protein (from *Rhizobium meliloti),* histone-like, DNA-binding protein (from *Pseudomonas putida),* histone-like, DNA-binding protein (from *Mycobacterium tuberculosis),* histone-like, DNA-binding protein (from *Mycobacterium leprae),* histone-like, DNA-binding protein (from *Zymomonas mobilis),* histone-like, DNA-binding protein (from *Yersinia pseudotuberculosis),* histone-like, DNA-binding protein (from *Mycobacterium bovis),* histone-like, DNA-binding protein (from *Mycoplasma hyopneumoniae),* histone-like, DNA-binding protein (from *Mycobacterium smegmatis),* histone-like, DNA-binding protein (from *Helicobacter pylori),* histone-like, DNA-binding protein (from *Aquifex aeolicus),* histone-like, DNA-binding protein (from *Agrobacterium tumefaciens),* histone-like, DNA-binding protein (from *Pseudomonas aeruginosa),* histone-like, DNA-binding protein (from *Helicobacter pylori),* histone-like, DNA-binding protein (from *Xanthomonas campestris),* histone-like, DNA-binding protein (from *Vibrio proteolyticus),* histone-like, DNA-binding protein (from *Streptomyces lividans),* histone-like, DNA-binding protein (from *Rickettsia prowazekii),* histone-like, DNA-binding protein (from *Streptomyces coelicolor),* histone-like, DNA-binding protein (from *Mycoplasma capricolum),* histone-like, DNA-binding protein (from *Borrelia burgdorferi),* histone-like, DNA-binding protein (from *Borrelia japonica),* histone-like, DNA-binding protein (from *Borrelia andersonii),* oder HTa (from *Thermoplasma acidophilum),* is employed.

13. The method according to one or more of the preceding claims,
**characterized in that**
the procaryotic HU-protein is modified in such a way that an increased or modified efficiency of uptake into the target cells occurs.

14. The method according to one or more of the preceding claims,
**characterized in that**
the prokaryotic HU-protein is modified in such a way that an increased release of the transferred nucleic acid, the nucleic acid analog or the nucleic acids or nucleic acid analogs or amino acid-containing substances from the endosomes of the target cells is achieved.

15. The method according to one or more of the preceding claims,
**characterized in that** the prokaryotic HU-protein is modified in such a way that the complex of nucleic acid, the nucleic acid analog or the nucleic acids or nucleic acid analogs or amino acid-containing substance and the prokaryotic HU-protein binds to a receptor molecule on the surface of the target cells.

16. The method according to one or more of the preceding claims,
**characterized in that**
prior to the incubation of the complex of nucleic acid, the nucleic acid analog or the nucleic acids or nucleic acid analogs or amino acid-containing substance and the prokaryotic HU-protein with the target cells, a wrapping of the complex with a liposome membrane or with polyethylene glycol occurs.

## Revendications

1. Procédé de transfert d' acides nucléiques et / ou d' analogues d' acides nucléiques , et ou de substances contenant des acides nucléiques et / ou des analogues d' acides nucléiques et / ou des acides aminés , dans des cellules procaryotiques et / ou eucaryotiques , selon lequel
- l' acide nucléique et / ou l' analogue d' acide nucléique et / ou la substance contenant des acides nucléiques et / ou des analogues d' acides nucléiques et / ou des acides aminés à transférer est mis en contact avec une protéine HU procaryotique , afin de former un complexe de l'acide nucléique et / ou de l' analogue d' acide nucléique, et / ou de la substance contenant des acides nucléiques et /ou des analogues d' acides nucléiques et / ou des acides aminés et de la protéine HU procaryotique , et
- enfin le complexe de l' acide nucléique et / ou de l' analogue d' acide nucléique , et / ou de la substance contenant des acides nucléiques et / ou des analogues d' acides nucléiques et / ou des acides aminés et de la protéine HU procaryotique est mis en contact avec les cellules-cible procaryotiques et / ou eucaryotiques , afin de réaliser un transfert du complexe dans les cellules .

2. Procédé conforme à la revendication 1 , **caractérisé en ce qu'** en tant qu' acide nucléique on transfère de l'ADN à simple brin ou à double brin , de l' AND sous forme de plasmides, de fragments de chromosomes , d' ARN antisens, de ribosomes, d' ARN catalytique , de nucléotides , d' AND chromosomal ou de mARN de codage .

3. Procédé conforme à la revendication 1 ou 2 , **caractérisé en ce qu'** en tant qu' acide nucléique on utilise un ANDN avec une séquence de codage qui est introduite pour expression dans les cellules cible .

4. Procédé conforme à l' une ou à plusieurs des revendications précédentes , **caractérisé en ce que** l' acide nucléique introduit est , de par sa mise en contact avec la protéine HU procaryotique , compacté et protégé contre une dégradation.

5. Procédé conforme à la revendication 1 , **caractérisé en ce qu'** en tant qu' analogue d' acide nucléique on transfère un ANP (acide nucléique peptide) .

6. Procédé conforme à une ou plusieurs des revendications 1 à 5 , **caractérisé en ce que** le complexe de l'acide nucléique et / ou de l'analogue d' acide nucléique et de la protéine HU procaryotique est réversible.

7. Procédé conforme à une ou plusieurs des revendications 1 à 5 pour le transfert d'ADN dans des cellules de plantes ou dans des cellules d' animaux ou dans des cellules humaines .

8. Procédé conforme à la revendication 1 , **caractérisé en ce qu'** en tant que substances contenant des acides aminés on transfère des protéines , des peptides , des hormones peptidiques , des enzymes , des domaines de protéines , des glycoprotéines , des agents actifs pharmaceutiques à base d' acides aminés , des peptides-hormones ou des lipoprotéines .

9. Procédé conforme à la revendication 8 , **caractérisé en ce que** la formation de complexe à partir de la substance contenant des acides aminés et de la protéine HU procaryotique a lieu par une liaison covalente ou non-covalente.

10. Procédé conforme à la revendication 8 ou 9 , **caractérisé en ce qu'** en tant que substances contenant des acides aminés on transfère la protéine fluorescente verte (PFV) .

11. Procédé conforme à l'une ou à plusieurs des revendications précédentes , **caractérisé en ce que** les protéines HU procaryotiques proviennent d' organismes cryophiles , mésophiles , thermophiles ou hyperthermophiles .

12. Procédé conforme à l'une ou à plusieurs des revendications précédentes , **caractérisé en ce qu'** en tant que protéine HU procaryotique on utilise TmHU (protéine HU *Thermotoga maritima)* , Sso7d (provenant de *Sulfolobus solfataricus* et *Sulfolobus acidocaldarius) ,* Ssh7 (provenant de *Sulfolobus shibatae)* , Sac7d (provenant de *Sulfolobus solfataricus* et *Sulfolobus acidocaldarius) ,* BstHU (provenant de *Bacilles stearothermophilus)* HU (provenant de *Escherichia coli*), IHF (provenant de *Escherichia coli*), Bsu HU (provenant de *Bacillus subtilis)* , SaHU (provenant de *Sulfolobus acidocaldarius)* , BbuHU (provenant de *Borrelia burgdorfen)* , BgaHU (provenant de *Borrelia garinii),* Baf-HU (provenant de *Borrelia afzelii)* , IHF (provenant de *Pseudomonas aeruginosa)* , la protéine se liant à l'ADN *(Chlamydia spp. ,* Hsa (provenant de *Staphylococcus aureus*) , RIHU (provenant *de Rhizobium leguminosarum) ,* HSI (provenant de *Streptomyces lividans)* , HCj (provenant de *Campylobacter jejuni) ,* HU (provenant de *Bacillus caldolyticus) ,* HU (provenant de *Bacillus caldotenax) ,* HU (provenant de *Bacillus globigii) ,* HCc (provenant de *Caulobacter crescentus)* la protéine se liant à l'ADN (provenant de *Deinococcus radiodurans) ,* HSa (provenant de *Sulfolobus acidocaldarius)* la protéine se liant à l'ADN et similaire à l' histone (provenant de *Streptococcus gordonii*)*,* la protéine se liant à l'ADN et similaire à l' histone (provenant de *Streptococcus mutans*)*,* la protéine se liant à l'ADN et similaire à l' histone (provenant de *Streptococcus pyogenes)* , la protéine se liant à l'ADN et similaire à l' histone (provenant de *Streptococcus thermophilus)* la protéine se liant à l' ADN et similaire à l' histone (provenant de *Haemophilus influenzae Rd)* , la protéine se liant à l'ADN et similaire à l' histone (provenant de *Listeria monocytogenes)* , la protéine se liant à l'ADN et similaire à l' histone (provenant de *Serratia marcescens)* la protéine se liant à l'ADN et similaire à l'histone (provenant de *Salmonella typhimurium),* la protéine se liant à l'ADN et similaire à l' histone (provenant de *Thermus aquaticus)* , la protéine se liant à l' ADN et similaire à l' histone (provenant de *Rhizobium meliloti)* , la protéine se liant à l'ADN et similaire à l' histone (provenant de *Pseudomonas putida)* , la protéine se liant à l' ADN et similaire à l' histone (provenant de *Mycobacterium tuberculosis)* , la protéine se liant à l' ADN et similaire à l' histone (provenant de *Mycobacterium leprae)* , la protéine se liant à l' ADN et similaire à l' histone (provenant de *Zymomonas mobilis)* , la protéine se liant à l' ADN et similaire à l' histone (provenant de *Yersinia pseudotuberculosis)* , la protéine se liant à l'ADN et similaire à l' histone (provenant de *Mycobacterium bovis*), la protéine se liant à l' ADN et similaire à l' histone (provenant *de Mycoptasma hyopneumoniae)* la protéine se liant à l' ADN et similaire à l' histone (provenant de *Mycobacterium smegmatis)* , la protéine se liant à l'ADN et similaire à l' histone (provenant de *Helicobacter pylon)* , la protéine se liant à l' ADN et similaire à l' histone (provenant de *Aquifex aeolicus)* , la protéine se liant à l' ADN et similaire à l' histone (provenant de *Agrobacterium tumefaciens)* la protéine se liant à l'ADN et similaire à l' histone (provenant de *Pseudomonas aeruginosa)* , la protéine se liant à l' ADN et similaire à l' histone (provenant de *Helicobacter pylon)* la protéine se liant à l'ADN et similaire à l' histone (provenant de *Xanthomonas campestris)* , la protéine se liant à l' ADN et similaire à l' histone (provenant de *Vibrio proteolyticus*)*,* la protéine se liant à l'ADN et similaire à l' histone (provenant de *Streptomyces lividans)* , la protéine se liant à l'ADN et similaire à l' histone (provenant de *Rickettsia prowazekii)* la protéine se liant à l' ADN et similaire à l' histone (provenant de *Streptomyces coelicolor)* la protéine se liant à l' ADN et similaire à l' histone (provenant de *Mycoplasma capricolum)* la protéine se liant à l' ADN et similaire à l' histone (provenant de *Borrelia burgdorferi*)*,* la protéine se liant à l' ADN et similaire à l' histone (provenant de *Borrelia japonica)* , la protéine se liant à l'ADN et similaire à l' histone (provenant de *Borrelia andersonii)* , ou HTa (provenant de *Thermoplasma acidophilum*).

13. Procédé conforme à l' une ou à plusieurs des revendications précédentes , **caractérisé en ce que** la protéine HU procaryotique est modifiée de telle façon qu' il s' ensuit une efficience d' introduction dans les cellules-cible accrue ou modifiée .

14. Procédé conforme à l'une ou à plusieurs des revendications précédentes , **caractérisé en ce que** la protéine HU procaryotique est modifiée de telle façon qu' on obtient une libération accrue , à partir des endosomes des cellules-cible , des acides nucléiques , des analogues d' acides nucléiques ou de la substance contenant des acides nucléiques ou des analogues d' acides nucléiques ou des acides aminés transférés.

15. Procédé conforme à l'une ou à plusieurs des revendications précédentes, **caractérisé en ce que** la protéine HU procaryotique est modifiée de telle façon que le complexe formé à partir d' acide nucléiques , d' analogues d' acide nucléiques , ou de la substance contenant des acides nucléiques ou des analogues d' acides nucléiques ou des acides aminés et de la protéine HU procaryotique se lie à une molécule réceptrice à la surface des cellules-cible .

16. Procédé conforme à l'une ou à plusieurs des revendications précédentes , **caractérisé en ce que** , avant l'incubation avec les cellules-cible du complexe formé à partir d' acide nucléiques , d' analogues d' acide nucléiques , ou de la substance contenant des acides nucléiques ou des analogues d' acides nucléiques ou des acides aminés et de la protéine HU procaryotique , a lieu un enrobage du complexe avec une membrane de liposomes ou avec du polyéthylène glycol.
